# EUROPEAN PATENT SPECIFICATION

(11) **EP 2 865 348 B1**
(45) Date of publication and mention of the grant of the patent: **08.03.2017**
(21) Application number: 13810198.5
(22) Date of filing: 18.06.2013
(51) Int. Cl.: A61B 18/12, A61B 17/34

(54) **MEDICAL WIRELESS POWER SUPPLY SYSTEM**
DRAHTLOSES MEDIZINISCHES STROMVERSORGUNGSSYSTEM
SYSTÈME D'ALIMENTATION ÉLECTRIQUE SANS FIL MÉDICAL

(30) Priority: 26.06.2012 JP 2012143300
(43) Date of publication of application: 29.04.2015
(73) Proprietor: Olympus Corporation, Tokyo 192-8507 (JP)
(72) Inventor: SUGIYAMA, Yuta, Tokyo 192-8507 (JP)
(74) Representative: Winter, Brandl, Fürniss, Hübner, Röss, Kaiser, Polte - Partnerschaft mbB
(86) International application number: PCT/JP2013/066735
(87) International publication number: WO 2014/002830

(56) References cited:
- EP-A1- 2 458 710
- JP-A- H11 128 242
- JP-A- 2000 254 134
- JP-A- 2004 208 922
- JP-A- 2011 030 317
- US-A- 3 358 256
- US-A- 5 849 020
- US-A1- 2010 052 431
- US-A1- 2010 081 875
- US-A1- 2011 139 877

## Description

### Technical Field

The present invention relates to a medical wireless power supply system and, more particularly, to a medical wireless power supply system that performs power supply to a medical instrument wirelessly.

### Background Art

In various instruments and apparatuses used in a medical field, those including a configuration for performing supply of electric power making use of an electromagnetic induction phenomenon have been proposed in recent years.

More specifically, for example, Japanese Patent Application Laid-Open Publication No. H11-128242 discloses a configuration for causing electromagnetic induction between a power transmission coil provided in a trocar and a power reception coil provided in a surgical operation instrument to thereby supply electric surgical operation energy to the surgical operation instrument inserted into the trocar. Further, US 5 849 020 A discloses that a cordless inductively coupled electrosurgical instrument is adapted to receive electrosurgical energy from a specially designed trocar. US 5 849 020 A also discloses a closure tube, a cannula tube, a central aperture, an inductor coil, an interior wall, an inductive electrosurgical adapter, an instrument inductor and an outer tube.

Furthermore, Document EP 2 458 710 A1 discloses a power transmission device with a power supply unit configured to supply power by a resonance frequency for magnetic resonance; a power transmission resonance coil capable of magnetic resonance with a power reception resonance coil in the resonance frequency, the power transmission resonance coil being configured to supply the power from the power supply unit as magnetic field energy caused by the magnetic resonance. Document US 2011/139877 A1 discloses an RFID tag comprising a magnetic core, a wire wrapped around the core, and an integrated circuit electrically connected to the wire. Document US 2010/081875 A1 discloses devices and methods for performing minimal access surgery to a surgical device comprising an insertable instrument attached to a trocar. Document US 2010/052431 A1 discloses an electromagnetic resonance non-contact power transmission device with a transmitter including a transmitter resonance element having a mechanism for discretely or continuously varying a resonant frequency, a transmitter excitation element coupled to the transmitter resonance element by electromagnetic induction.

However, according to the configuration disclosed in Japanese Patent Application Laid-Open Publication No. H11-128242, since the supply of the electric power is performed making use of the electromagnetic induction phenomenon, a problem occurs in that a positional relation between the power transmission coil and the power reception coil has a relatively large influence on transmission efficiency of the electric power. As a result, according to the configuration disclosed in Japanese Patent Application Laid-Open Publication No. H11-128242, for example, depending on positions where the power transmission coil and the power reception coil are arranged, a situation occurs in which transmission efficiency of electric power supplied from the trocar to the surgical operation instrument does not reach predetermined transmission efficiency and sufficient electric surgical operation energy is not supplied to treatment performed using the surgical operation instrument.

The present invention has been devised in view of the circumstances explained above and it is an object of the present invention to provide a medical wireless power supply system capable of improving a degree of freedom of portions related to power transmission and power reception while preventing deterioration in transmission efficiency of electric power as much as possible.

### Disclosure of Invention

### Means for Solving the Problem

A medical wireless power supply system according to an aspect of the present invention includes: a trocar; a plurality of medical instruments including a cylindrical insertion portion insertable into an insertion hole of the trocar; a plurality of power transmission coils provided in a state in which at least a part thereof is covered with a nonconductive member in the vicinity of the insertion hole of the trocar; a plurality of power transmission coil units provided in the trocar, the power transmission coil units each including a respective one of the plurality of power transmission coils and configured to be capable of transmitting electric power supplied from the trocar to the medical instruments by respectively resonating at predetermined resonance frequencies differing from each other; a power reception coil in each of the plurality of medical instruments provided in a state in which at least a part thereof is covered with a nonconductive member on an inside of the insertion portion; and a power reception coil unit provided in each of the medical instruments, each power reception coil unit including the power reception coil and configured to be capable of receiving electric power transmitted from the trocar by resonating at a predetermined resonance frequency that coincides with a respective one of the predetermined resonance frequencies of the plurality of power transmission coil units.

### Brief Description of the Drawings

Fig. 1A is a diagram showing an external appearance of a trocar according to a first example;
Fig. 1B is a diagram showing a configuration of a main part of the trocar according to the first example;
Fig. 2 is a diagram showing a configuration of a main part of a bipolar electric knife according to the first example;
Fig. 3 is a diagram showing a distal end portion of the bipolar electric knife in Fig. 2 in enlargement;
Fig. 4 is a diagram showing an example of a state in which the bipolar electric knife is inserted through the trocar;
Fig. 5 is a diagram showing a configuration of a main part of a medical wireless power supply system including the trocar and the bipolar electric knife;
Fig. 6A is a diagram showing an external appearance of a trocar according to a first modification of the first example;
Fig. 6B is a diagram showing a configuration of a main part of the trocar according to the first modification of the first example;
Fig. 7A is a diagram showing an external appearance of a trocar according to a second modification of the first example;
Fig. 7B is a diagram showing an internal configuration of a main part of the trocar according to the second modification of the first example;
Fig. 7C is a diagram showing a case in which a power transmission coil unit is attached to a trocar main body portion in Fig. 7B;
Fig. 8A is a diagram showing an external appearance of a trocar according to a third modification of the first example;
Fig. 8B is a diagram showing an internal configuration of a main part of the trocar according to the third modification of the first example;
Fig. 9 is a diagram showing a configuration of a main part of a trocar according to a fourth modification of the first example;
Fig. 10 is a diagram showing a configuration of a main part of a bipolar electric knife according to a fifth modification of the first example;
Fig. 11 is a diagram showing a configuration of a main part of a bipolar electric knife according to a sixth modification of the first example;
Fig. 12A is a diagram showing an external appearance of a bipolar electric knife according to a second example;
Fig. 12B is a sectional view showing a configuration of an insertion portion of the bipolar electric knife in Fig. 12A;
Fig. 13A is a diagram showing an external appearance of a bipolar electric knife according to a first modification of the second example;
Fig. 13B is a sectional view showing a configuration of an insertion portion of the bipolar electric knife in Fig. 13A;
Fig. 14 is a sectional view showing a configuration of an insertion portion according to a second modification of the second example;
Fig. 15 is a sectional view showing a configuration of an insertion portion according to a third modification of the second example;
Fig. 16 is a sectional view showing a configuration of an insertion portion according to a fourth modification of the second example;
Fig. 17A is a sectional view showing a configuration of an insertion portion according to a fifth modification of the second example;
Fig. 17B is a sectional view showing a configuration of an insertion portion according to a sixth modification of the second example;
Fig. 18A is a diagram showing a configuration of a main part of a bipolar electric knife according to a third example;
Fig. 18B is a diagram showing a part of an insertion portion of the bipolar electric knife in Fig. 18A in enlargement;
Fig. 19A is a diagram showing a configuration of a trocar according to a fourth example;
Fig. 19B is a diagram showing an example of an aspect of use of the trocar in Fig. 19A;
Fig. 20 is a diagram showing a configuration of a trocar according to a modification of the fourth example;
Fig. 21 is a diagram showing a configuration of a trocar according to a fifth example;
Fig. 22 is a diagram showing a configuration of a trocar according to a modification of the fifth example;
Fig. 23 is a diagram showing a configuration of a trocar according to a sixth example, which is an embodiment of the invention;
Fig. 24 is a diagram showing a configuration of a trocar according to a modification of the sixth example, which is an embodiment of the invention;
Fig. 25A is a diagram showing an external appearance of a trocar according to a seventh example, which is an embodiment of the invention;
Fig. 25B is a diagram showing an internal configuration of a main part of the trocar according to the seventh example, which is an embodiment of the invention;
Fig. 26A is a diagram showing an external appearance of a trocar according to a first modification of the seventh example, which is an embodiment of the invention;
Fig. 26B is a diagram showing an internal configuration of a main part of the trocar according to the first modification of the seventh example, which is an embodiment of the invention;
Fig. 27A is a diagram showing an external appearance of a trocar according to a second modification of the seventh example;
Fig. 27B is a diagram showing an internal configuration of a main part of the trocar according to the second modification of the seventh example;
Fig. 28 is a diagram showing a configuration of a power transmission coil unit according to an eighth example;
Fig. 29 is a diagram showing an example of a configuration in which a power transmission coil unit capable of resonating in parallel is provided in a trocar;
Fig. 30 is a diagram showing an example of a configuration in which a power reception coil unit capable of resonating in parallel is provided in a bipolar electric knife; and
Fig. 31 is a diagram showing an example of a component that can be incorporated in the power transmission coil unit.

### Best Mode for Carrying Out the Invention

Examples are explained below with reference to the drawings. The sixth and seventh example are embodiments of the invention, as well as the first modification of the seventh example.

### (First Example)

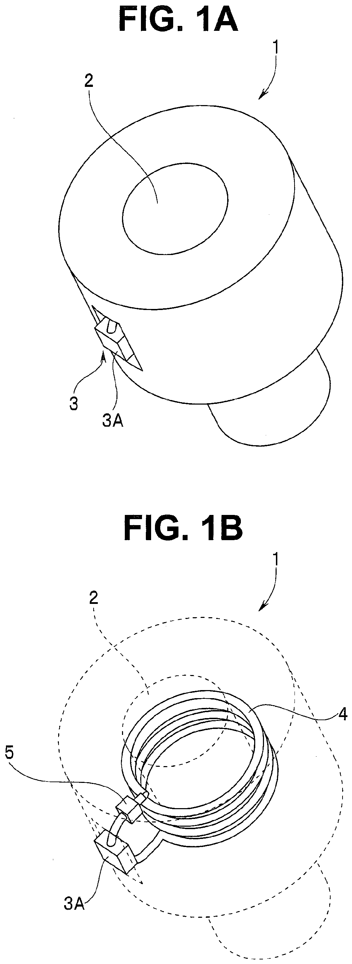

Fig. 1A to Fig. 11 relate to a first example. Fig. 1A is a diagram showing an external appearance of a trocar according to the first example.

Fig. 1B is a diagram showing a configuration of a main part of the trocar according to the first example.

As shown in Fig. 1A, a trocar 1 is formed to have a substantially convex shape. The trocar 1 includes an insertion hole 2 formed as a hole having a dimension for enabling a treatment instrument and the like to be inserted and a connection terminal portion 3 formed such that a power transmission cable 24 explained below can be detachably connected thereto.

As shown in Fig. 1B, on an inside of the trocar 1, a power transmission coil 4 wound along an outer circumference portion of the insertion hole 2 and a power transmission capacitor 5 connected in series to an electric terminal 3A provided in the connection terminal portion 3 and the power transmission coil 4 are provided.

Peripheries of the power transmission coil 4 and the power transmission capacitor 5 on the inside of the trocar 1 are covered with insulating members such as resin. Note that, according to the present example, only a part of the power transmission coil 4 may be configured to be covered with the insulating member such as resin.

The power transmission coil 4 is wound to include, on the inside of the trocar 1 and in the outer circumference portion of the insertion hole 2, a winding axis parallel to an axis in an inserting direction of the insertion hole 2 (hereinafter also referred to as insertion axis) (or coinciding with the insertion axis) and generally cover a vicinity of an opening portion (an upper opening portion) on an inlet side of the insertion hole 2. One end portion of the power transmission coil 4 is connected to the power transmission capacitor 5. The other end portion of the power transmission coil 4 is connected to the electric terminal 3A.

Further, in the trocar 1, inductance of the power transmission coil 4 and capacitance of the power transmission capacitor 5 are respectively set such that the power transmission coil 4 and the power transmission capacitor 5 have a predetermined series resonance frequency (e.g., 13.56 MHz) that coincides with a series resonance frequency of a power reception coil unit incorporated in a bipolar electric knife 11 explained below.

That is, on the inside of the trocar 1, a power transmission coil unit functioning as a series resonance circuit including the power transmission coil 4 and the power transmission capacitor 5 is provided.

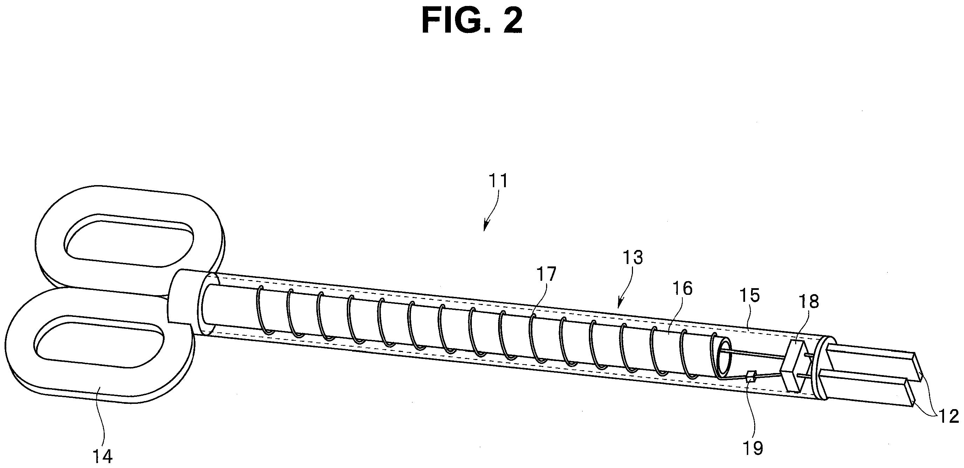

Fig. 2 is a diagram showing a configuration of a main part of a bipolar electric knife according to the first example.

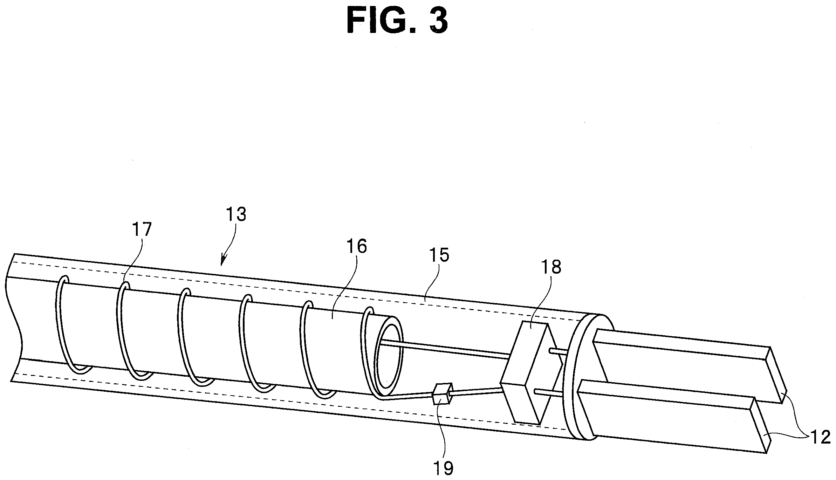

Fig. 3 is a diagram showing a distal end portion of the bipolar electric knife in Fig. 2 in enlargement.

As shown in Fig. 2, a bipolar electric knife 11 includes, at a distal end portion, treatment electrodes 12 capable of applying a high-frequency current for performing treatment such as dissection or coagulation to a living tissue, includes, in a halfway portion, an insertion portion 13 having an elongated shape insertable into the insertion hole 2 of the trocar 1, and includes, at a rear end portion, an operation portion 14 that enables operation related to opening and closing motions of the treatment electrodes 12 to be manually performed.

As shown in Fig. 2, the bipolar electric knife 11 is configured such that water tightness of an inside of the insertion portion 13 is kept by a cylindrical outer cylinder portion 15 formed by an insulating member such as resin.

Further, as shown in Fig. 2 and Fig. 3, on an inside of the outer cylinder portion 15, a cylindrical inner cylinder portion 16 formed by an insulating member such as resin, a power reception coil 17 wound along an outer circumference portion of the inner cylinder portion 16, a conversion circuit 18 capable of converting a waveform of an alternating current received in the power reception coil 17 into a waveform suitable for treatment (such as dissection or coagulation) and driving the treatment electrodes 12, and a power reception capacitor 19 connected in series to the power reception coil 17 and the conversion circuit 18 are provided.

The power reception coil 17 is wound to include, on the inside of the outer cylinder portion 15 and in the outer circumference portion of the inner cylinder portion 16, a winding axis parallel to (or coinciding with) a major axis of the insertion portion 13 and generally cover a portion ranging from a vicinity of the treatment electrodes 12 to a vicinity of the operation portion 14 (a portion ranging from a distal end side to a rear end side of the insertion portion 13). One end portion of the power reception coil 17 is connected to the power reception capacitor 19. The other end portion of the power reception coil 17 is connected to the conversion circuit 18.

Further, in the bipolar electric knife 11, inductance of the power reception coil 17 and capacitance of the power reception capacitor 19 are respectively set such that the power reception coil 17 and the power reception capacitor 19 have predetermined series resonance frequency (e.g., 13.56 MHz) that coincides with the series resonance frequency of the power transmission coil unit incorporated in the trocar 1.

That is, on an inside of the bipolar electric knife 11, a power reception coil unit functioning as a series resonance circuit including the power reception coil 17 and the power reception capacitor 19 is provided.

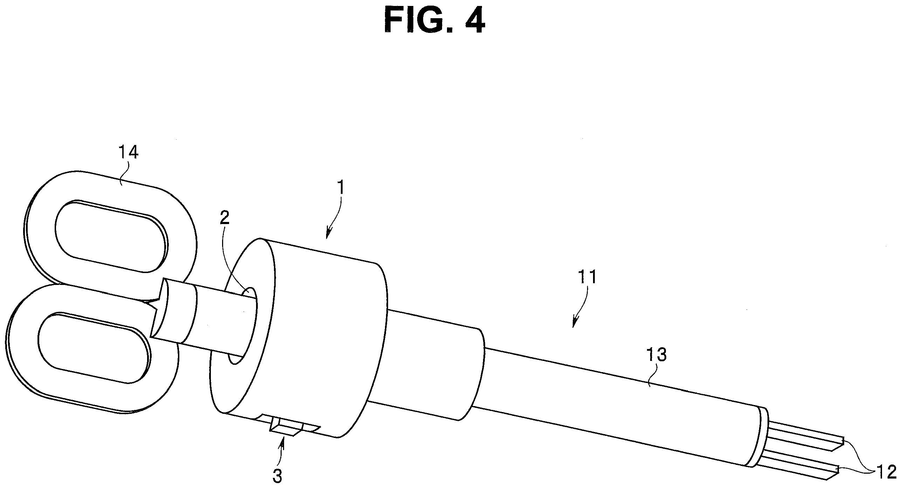

For example, as shown in Fig. 4, the bipolar electric knife 11 is used in a state in which the insertion portion 13 is inserted through the insertion hole 2 of the trocar 1. Fig. 4 is a diagram showing an example of a state in which the bipolar electric knife is inserted through the trocar.

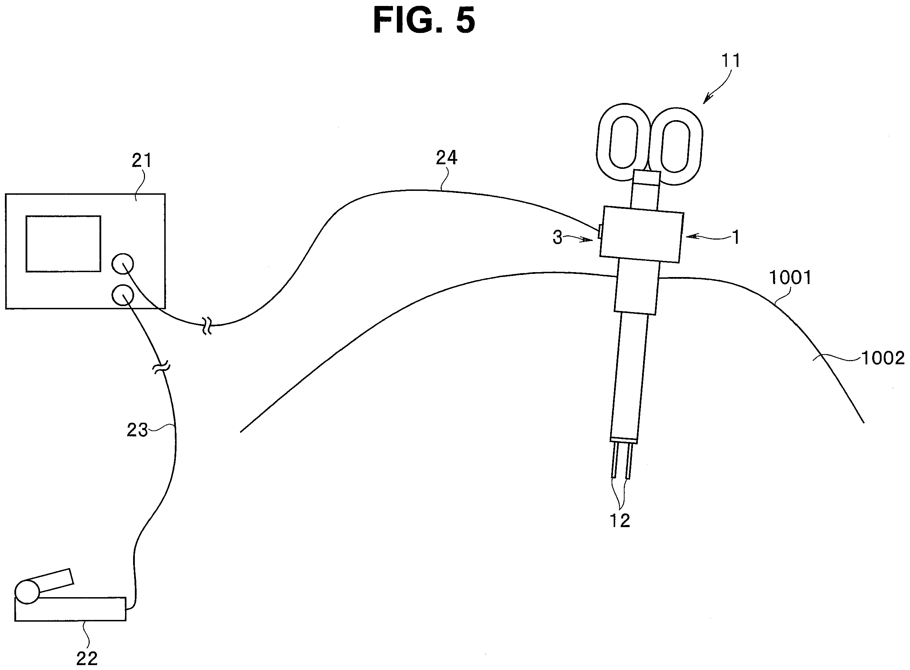

A specific aspect of use of a medical wireless power supply system including the trocar 1 and the bipolar electric knife 11 is explained. Fig. 5 is a diagram showing a configuration of a main part of the medical wireless power supply system including the trocar and the bipolar electric knife according to the first example.

First, for example, as shown in Fig. 5, a surgeon or the like punctures a body wall 1001 of a subject with the trocar 1, connects, using a signal cable 23, a power supply device 21 that supplies alternating-current power to the trocar 1 and a foot switch 22 capable of outputting an instruction signal for turning on or off power supply from the power supply device 21 to the trocar 1, and connects the power supply device 21 and the connection terminal portion 3 of the trocar 1 using a power transmission cable 24.

Thereafter, the surgeon or the like inserts the bipolar electric knife 11 into a body cavity 1002 of the subject via the trocar 1 set in the body wall 1001 of the subject.

When the surgeon or the like confirms, for example, through observation of an endoscopic image, visual observation, or the like, that the treatment electrodes 12 reach a vicinity of a treatment target region of the body cavity 1002, the surgeon or the like operates the foot switch 22 to thereby turn on power supply from the power supply device 21 to the trocar 1.

On the other hand, the power supply device 21 supplies, on the basis of an instruction outputted according to the operation of the foot switch 22 by the surgeon or the like, for example, alternating-current power having a frequency that coincides with a resonance frequency of the power transmission coil unit of the trocar 1. According to the supply of such alternating-current power, a magnetic field resonance phenomenon occurs between the power transmission coil unit of the trocar 1 and the power reception coil unit of the bipolar electric knife 11. Power transmission from the power transmission coil 4 to the power reception coil 17 is performed. The alternating-current power received in the power reception coil 17 is used as driving power for the treatment electrodes 12.

Note that the frequency of the alternating-current power supplied from the power supply device 21 to the trocar 1 may be set to any frequency different from the resonance frequency of the power transmission coil unit of the trocar 1 as long as transmission efficiency equal to or higher than a predetermined value can be secured in the power transmission from the power transmission coil 4 to the power reception coil 17.

The power supply device 21 is not limited to a power supply device including a configuration capable of receiving, via the signal cable 23 connected to the foot switch 22, an instruction signal for turning on or off the power supply to the trocar 1. More specifically, the power supply device 21 may be a power supply device including a configuration capable of detecting, for example, when a wireless signal corresponding to operation of a power switch (not shown in the figure) provided in the operation portion 14 is outputted from the bipolar electric knife 11, on the basis of the wireless signal that the power supply to the trocar 1 is turned on or off.

With the medical wireless power supply system including the trocar 1 and the bipolar electric knife 11, since the medical wireless power supply system is used in the aspect explained above, it is possible to set arrangement positions of the power transmission coil 4 and the power reception coil 17 close to each other and keep directions of the power transmission coil 4 and the power reception coil 17 substantially fixed. As a result, with the medical wireless power supply system including the trocar 1 and the bipolar electric knife 11, it is possible to perform power supply at relatively high transmission efficiency while making use of the magnetic field resonance phenomenon.

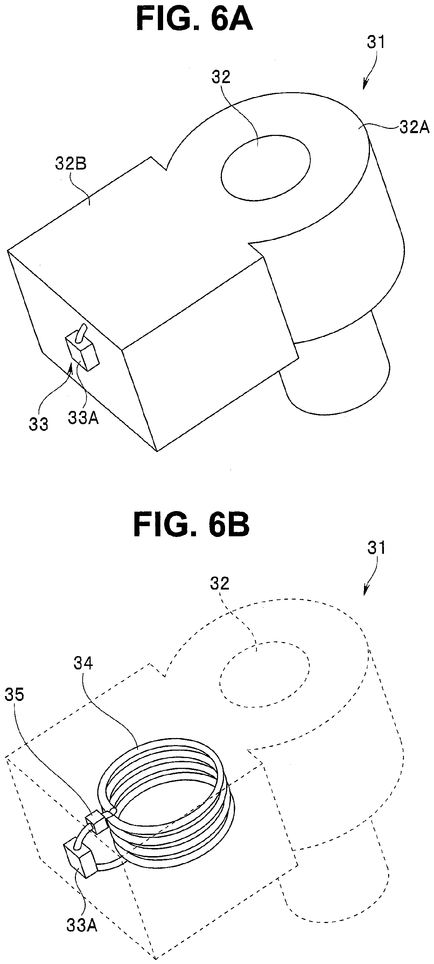

Note that, in the present example, instead of the trocar 1 including the configuration shown in Fig. 1A and Fig. 1B, for example, a trocar 31 including a configuration shown in Fig. 6A and Fig. 6B may be used. Fig. 6A is a diagram showing an external appearance of a trocar according to a first modification of the first example.

Fig. 6B is a diagram showing a configuration of a main part of the trocar according to the first modification of the first example.

As shown in Fig. 6A, the trocar 31 includes a trocar main body portion 32A having a substantially convex shape and a box-shaped power transmission coil unit 32B integrally provided in a side portion of the trocar main body portion 32A. As shown in Fig. 6A, the trocar main body portion 32A includes an insertion hole 32 formed as a hole having a dimension for enabling a treatment instrument and the like to be inserted. Further, as shown in Fig. 6A, the power transmission coil unit 32B includes a connection terminal portion 33 formed such that the power transmission cable 24 can be detachably connected thereto.

As shown in Fig. 6B, on an inside of the power transmission coil unit 32B, a power transmission coil 34 wound to include a winding axis parallel to an insertion axis of the insertion hole 32 and a power transmission capacitor 35 connected in series to an electric terminal 33A provided in the connection terminal portion 33 and the power transmission coil 34 are provided. As shown in Fig. 6B, one end portion of the power transmission coil 34 is connected to the power transmission capacitor 35 and the other end portion of the power transmission coil 34 is connected to the electric terminal 33A.

Further, peripheries of the power transmission coil 34 and the power transmission capacitor 35 on the inside of the power transmission coil unit 32B are covered with insulating members such as resin.

On the other hand, in the trocar 31, inductance of the power transmission coil 34 and capacitance of the power transmission capacitor 35 are respectively set such that the power transmission coil 34 and the power transmission capacitor 35 have a predetermined series resonance frequency (e.g., 13.56 MHz) that coincides with the series resonance frequency of the power reception coil unit incorporated in the bipolar electric knife 11.

Even when a medical wireless power supply system including the trocar 31 including the configuration explained above and the bipolar electric knife 11 including the configuration explained above is used, it is possible to perform power supply at relatively high transmission efficiency while making use of the magnetic field resonance phenomenon.

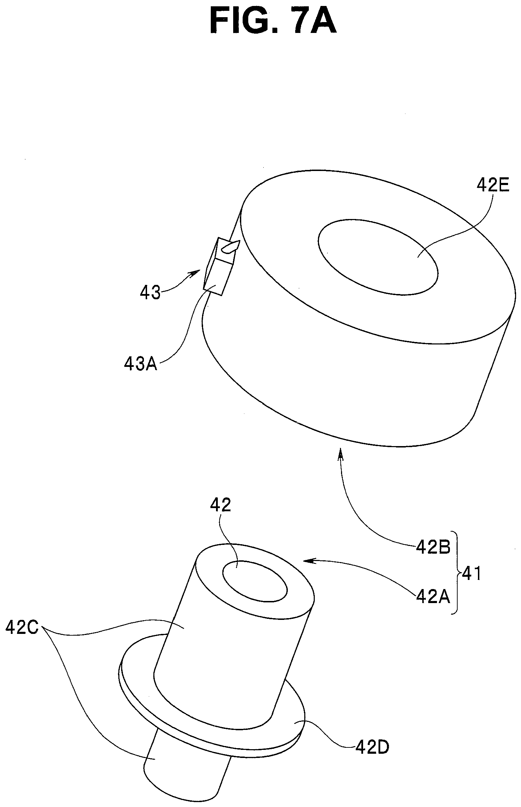

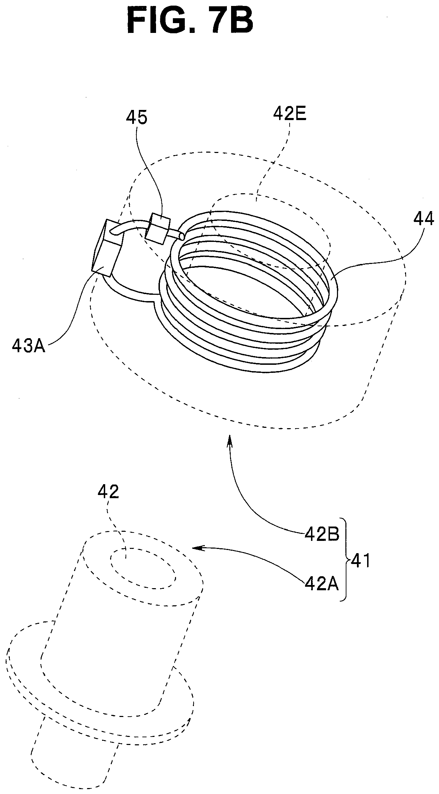

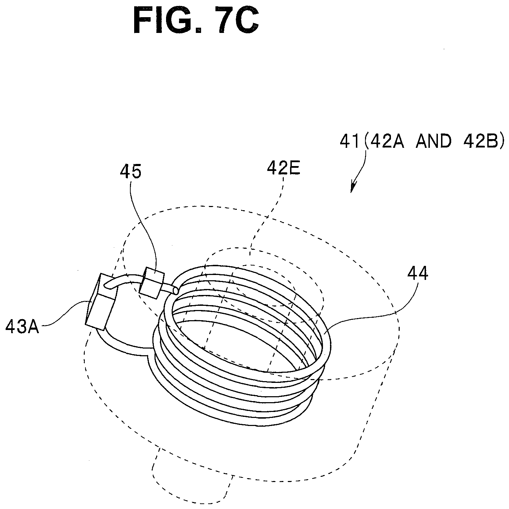

In the present example, instead of the trocar 1 including the configuration shown in Fig. 1A and Fig. 1B, for example, a trocar 41 including a configuration shown in Fig. 7A, Fig. 7B, and Fig. 7C may be used. Fig. 7A is a diagram showing an external appearance of a trocar according to a second modification of the first example.

Fig. 7B is a diagram showing an internal configuration of a main part of the trocar according to the second modification of the first example.

Fig. 7C is a diagram showing a case in which a power transmission coil unit is attached to a trocar main body portion in Fig. 7B.

As shown in Fig. 7A, the trocar 41 includes a trocar main body portion 42A and a cylindrical power transmission coil unit 42B detachably attachable to the trocar main body portion 42A. As shown in Fig. 7A, the trocar main body portion 42A includes a cylinder portion 42C including an insertion hole 42 formed as a hole having a dimension for enabling a treatment instrument and the like to be inserted and a flange portion 42D formed along an outer circumference of the cylinder portion 42C. Further, as shown in Fig. 7A, the power transmission coil unit 42B includes a fitting hole 42E formed as a hole having a dimension for enabling the cylinder portion 42C to be inserted through to come into contact with the flange portion 42D and a connection terminal portion 43 formed such that the power transmission cable 24 can be detachably connected thereto.

As shown in Fig. 7B, on an inside of the power transmission coil unit 42B, a power transmission coil 44 wound along an outer circumference portion of the fitting hole 42E and a power transmission capacitor 45 connected in series to an electric terminal 43A provided in the connection terminal portion 43 and the power transmission coil 44 are provided. As shown in Fig. 7B, one end portion of the power transmission coil 44 is connected to the power transmission capacitor 45 and the other end portion of the power transmission coil 44 is connected to the electric terminal 43A.

Further, peripheries of the power transmission coil 44 and the power transmission capacitor 45 on the inside of the power transmission coil unit 42B are covered with insulating members such as resin.

On the other hand, the power transmission coil unit 42B is attached to the trocar main body portion 42A in an aspect shown in Fig. 7C by inserting the cylinder portion 42C through the fitting hole 42E. Since the trocar main body portion 42A and the power transmission coil unit 42B are attached in this way, an insertion axis of the insertion hole 42 and a winding axis of the power transmission coil 44 are parallel to each other. The power transmission coil 44 is arranged to be located in a vicinity of an opening portion on an inlet side of the insertion hole 42.

In the power transmission coil unit 42B, inductance of the power transmission coil 44 and capacitance of the power transmission capacitor 45 are respectively set such that the power transmission coil unit 42B has a predetermined series resonance frequency (e.g., 13.56 MHz) that coincides with the series resonance frequency of the power reception coil unit incorporated in the bipolar electric knife 11.

Even when a medical wireless power supply system including the trocar 41 including the configuration explained above and the bipolar electric knife 11 including the configuration explained above is used, it is possible to perform power supply at relatively high transmission efficiency while making use of the magnetic field resonance phenomenon.

With the trocar 41 including the configuration explained above, for example, according to necessity of supply of electric power to a medical instrument used in a state in which the trocar 41 is inserted through the insertion hole 42, the power transmission coil unit 42B can be attached to the trocar main body portion 42A (or detached from the trocar main body portion 42A) and used.

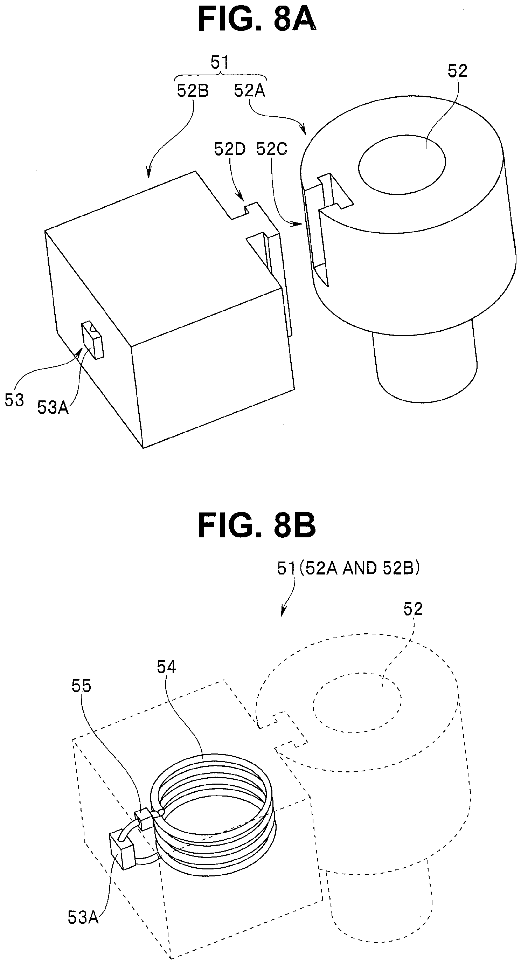

In the present example, instead of the trocar 1 including the configuration shown in Fig. 1A and Fig. 1B, for example, a trocar 51 including a configuration shown in Fig. 8A and Fig. 8B may be used. Fig. 8A is a diagram showing an external appearance of a trocar according to a third modification of the first example.

Fig. 8B is a diagram showing an internal configuration of a main part of the trocar according to the third modification of the first example.

As shown in Fig. 8A, the trocar 51 includes a trocar main body portion 52A and a box-shaped power transmission coil unit 52B detachably attachable to the trocar main body portion 52A. As shown in Fig. 8A, the trocar main body portion 52A includes an insertion hole 52 formed as a hole having a dimension for enabling a treatment instrument and the like to be inserted and a groove portion 52C formed around the insertion hole 52. Further, as shown in Fig. 8A, the power transmission coil unit 52B includes a projecting portion 52D formed to have a shape that can be fit in the groove portion 52C and a connection terminal portion 53 formed such that the power transmission cable 24 can be detachably connected thereto.

As shown in Fig. 8B, on an inside of the power transmission coil unit 52B, a power transmission coil 54 wound to include a winding axis parallel to an insertion axis of the insertion hole 52 when the projecting portion 52D is fit in the groove portion 52C (when the power transmission coil unit 52B is attached to the trocar main body portion 52A) and a power transmission capacitor 55 connected in series to an electric terminal 53A provided in the connection terminal portion 53 and the power transmission coil 54 are provided. As shown in Fig. 8B, one end portion of the power transmission coil 54 is connected to the power transmission capacitor 55 and the other end portion of the power transmission coil 54 is connected to the electric terminal 53A.

In the power transmission coil unit 52B, inductance of the power transmission coil 54 and capacitance of the power transmission capacitor 55 are respectively set such that the power transmission coil unit 52B has a predetermined series resonance frequency (e.g., 13.56 MHz) that coincides with the series resonance frequency of the power reception coil unit incorporated in the bipolar electric knife 11.

Even when a medical wireless power supply system including the trocar 51 including the configuration explained above and the bipolar electric knife 11 including the configuration explained above is used, it is possible to perform power supply at relatively high transmission efficiency while making use of the magnetic field resonance phenomenon.

With the trocar 51 including the configuration explained above, for example, according to necessity of supply of electric power to a medical instrument used in a state in which the medical instrument is inserted through the insertion hole 52, the power transmission coil unit 52B can be attached to the trocar main body portion 52A (or detached from the trocar main body portion 52A) and used.

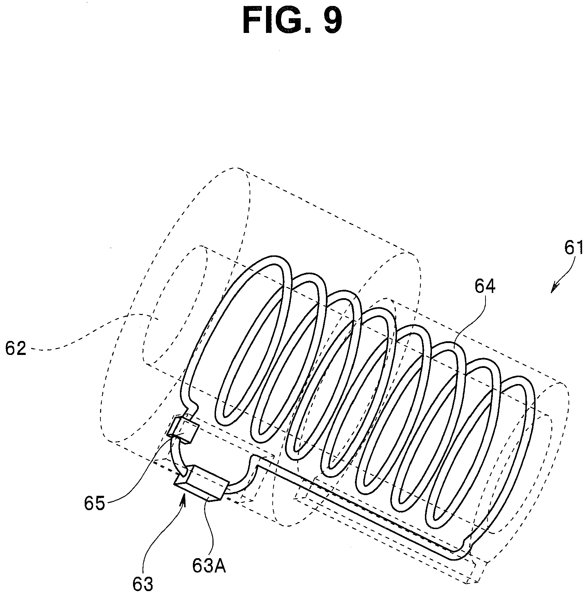

In the present example, instead of the trocar 1 including the configuration shown in Fig. 1A and Fig. 1B, for example, a trocar 61 including a configuration shown in Fig. 9 may be used. Fig. 9 is a diagram showing a configuration of a main part of a trocar according to a fourth modification of the first example.

As shown in Fig. 9, the trocar 61 is formed to have a substantially convex shape. The trocar 61 includes an insertion hole 62 formed as a hole having a dimension for enabling a treatment instrument and the like to be inserted and a connection terminal portion 63 formed such that the power transmission cable 24 can be detachably connected thereto.

As shown in Fig. 9, on an inside of the trocar 61, a power transmission coil 64 wound along an outer circumference portion of the insertion hole 62 and a power transmission capacitor 65 connected in series to an electric terminal 63A provided in the connection terminal portion 63 and the power transmission coil 64 are provided.

Peripheries of the power transmission coil 64 and the power transmission capacitor 65 on the inside of the trocar 61 are covered with insulating members such as resin.

The power transmission coil 64 is wound to include, on the inside of the trocar 61 and in the outer circumference portion of the insertion hole 62, a winding axis parallel to an insertion axis of the insertion hole 62 and generally cover a portion ranging from a vicinity of an opening portion on an inlet side of the insertion hole 62 to a vicinity of an opening portion (a lower opening portion) on an outlet side. One end portion of the power transmission coil 64 is connected to the power transmission capacitor 65. The other end portion of the power transmission coil 64 is connected to the electric terminal 63A.

On the other hand, in the trocar 61, inductance of the power transmission coil 64 and capacitance of the power transmission capacitor 65 are respectively set such that the power transmission coil 64 and the power transmission capacitor 65 have a predetermined series resonance frequency (e.g., 13.56 MHz) that coincides with the series resonance frequency of the power reception coil unit incorporated in the bipolar electric knife 11.

That is, on the inside of the trocar 61, a power transmission coil unit functioning as a series resonance circuit including the power transmission coil 64 and the power transmission capacitor 65 is provided.

Even when a medical wireless power supply system including the trocar 61 including the configuration explained above and the bipolar electric knife 11 including the configuration explained above is used, it is possible to perform power supply at relatively high transmission efficiency while making use of the magnetic field resonance phenomenon.

With the trocar 61 including the configuration explained above, the power transmission coil 64 is provided in the portion ranging from the vicinity of the opening portion on the inlet side of the insertion hole 62 to the vicinity of the opening portion on the outlet side. Therefore, it is easy to improve transmission efficiency in power supply performed making use of the magnetic field resonance phenomenon.

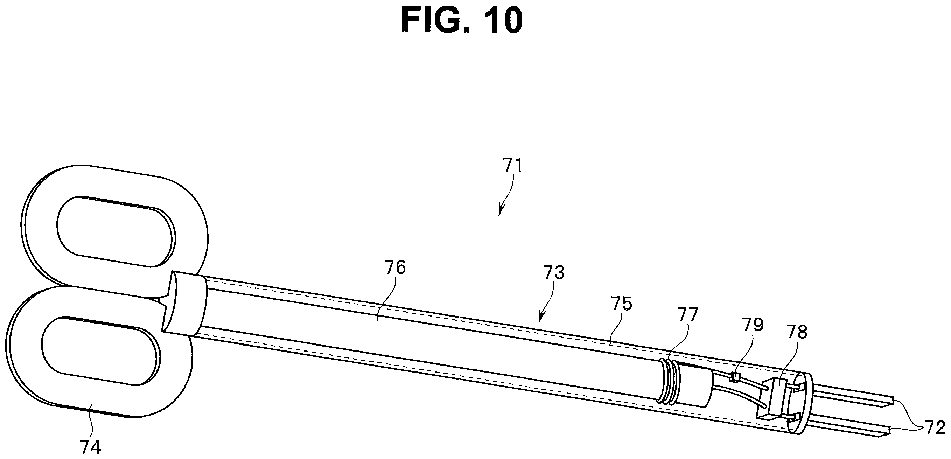

Note that, in the present example, instead of the bipolar electric knife 11 including the configuration shown in Fig. 2 and Fig. 3, for example, a bipolar electric knife 71 including a configuration shown in Fig. 10 may be used. Fig. 10 is a diagram showing a configuration of a main part of a bipolar electric knife according to a fifth modification of the first example.

As shown in Fig. 10, the bipolar electric knife 71 includes, at a distal end portion, treatment electrodes 72 capable of applying a high-frequency current for performing treatment such as dissection or coagulation to a living tissue, includes, in a halfway portion, an insertion portion 73 having an elongated shape insertable into the insertion holes of the respective trocars explained above, and includes, at a rear end portion, an operation portion 74 that enables operation related to opening and closing motions of the treatment electrodes 72 to be manually performed.

As shown in Fig. 10, the bipolar electric knife 71 is configured such that water tightness of an inside of the insertion portion 73 is kept by a cylindrical outer cylinder portion 75 formed by an insulating member such as resin.

Further, as shown in Fig. 10, on an inside of the outer cylinder portion 75, a cylindrical inner cylinder portion 76 formed by an insulating member such as resin, a power reception coil 77 wound along an outer circumference portion of the inner cylinder portion 76, a conversion circuit 78 capable of converting a waveform of an alternating current received in the power reception coil 77 into a waveform suitable for treatment (such as dissection or coagulation) and driving the treatment electrodes 72, and a power reception capacitor 79 connected in series to the power reception coil 77 and the conversion circuit 78 are provided.

The power reception coil 77 is wound to include, on the inside of the outer cylinder portion 75 and in the outer circumference portion of the inner cylinder portion 76, a winding axis parallel to a major axis of the insertion portion 73 and generally cover a vicinity of the treatment electrodes 72. One end portion of the power reception coil 77 is connected to the power reception capacitor 79. The other end portion of the power reception coil 77 is connected to the conversion circuit 78.

On the other hand, in the bipolar electric knife 71, inductance of the power reception coil 77 and capacitance of the power reception capacitor 79 are respectively set such that the power reception coil 77 and the power reception capacitor 79 have a predetermined series resonance frequency (e.g., 13.56 MHz) that coincides with the series resonance frequency of the power transmission coil units incorporated in the respective trocars explained above.

That is, on an inside of the bipolar electric knife 71, a power reception coil unit functioning as a series resonance circuit including the power reception coil 77 and the power reception capacitor 79 is provided.

Even when a medical wireless power supply system including the bipolar electric knife 71 including the configuration explained above and one of the respective trocars explained above is used, it is possible to perform power supply at relatively high transmission efficiency while making use of the magnetic field resonance phenomenon.

With the bipolar electric knife 71 including the configuration explained above, the power reception coil 77 can be reduced in size (as illustrated in Fig. 10). Therefore, it is possible to improve assembly accuracy in manufacturing of the insertion portion 73.

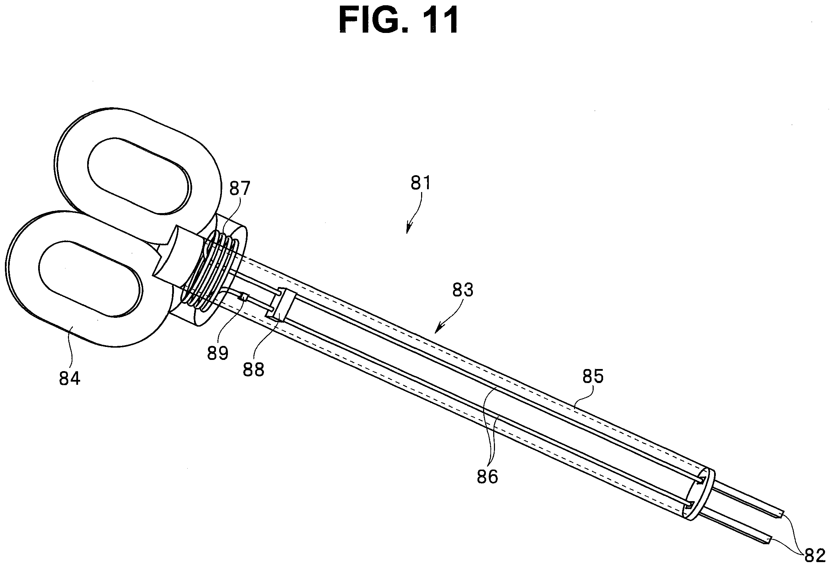

Note that, in the present example, instead of the bipolar electric knife 11 including the configuration shown in Fig. 2 and Fig. 3, for example, a bipolar electric knife 81 including a configuration shown in Fig. 11 may be used. Fig. 11 is a diagram showing a configuration of a main part of a bipolar electric knife according to a sixth modification of the first example.

As shown in Fig. 11, the bipolar electric knife 81 includes, at a distal end portion, treatment electrodes 82 capable of applying a high-frequency current for performing treatment such as dissection or coagulation to a living tissue, includes, in a halfway portion, an insertion portion 83 having an elongated shape insertable into the insertion holes of the respective trocars explained above, and includes, at a rear end portion, an operation portion 84 that enables operation related to opening and closing motions of the treatment electrodes 82 to be manually performed.

As shown in Fig. 11, the bipolar electric knife 81 is configured such that water tightness of an inside of the insertion portion 83 is kept by a cylindrical outer cylinder portion 85 formed by an insulating member such as resin.

Further, as shown in Fig. 11, on an inside of the outer cylinder portion 85, conductive wires 86 respectively connected to the two treatment electrodes 82, a power reception coil 87 provided in a vicinity of the operation portion 84, a conversion circuit 88 capable of converting a waveform of an alternating current received in the power reception coil 87 into a waveform suitable for treatment (such as dissection or coagulation) and outputting the alternating-current to the conductive wires 86, and a power reception capacitor 89 connected in series to the power reception coil 87 and the conversion circuit 88 are provided.

The power reception coil 87 is wound to include, on the inside of the outer cylinder portion 85 and in the vicinity of the operation portion 84, a winding axis parallel to a major axis of the insertion portion 83 and provided in a position where the power reception coil 87 is arranged near an opening portion on an inlet side of the insertion hole when the insertion portion 83 is inserted through the insertion hole of any one of the respective trocars explained above. One end portion of the power reception coil 87 is connected to the power reception capacitor 89. The other end portion of the power reception coil 87 is connected to the conversion circuit 88.

On the other hand, in the bipolar electric knife 81, inductance of the power reception coil 87 and capacitance of the power reception capacitor 89 are respectively set such that the power reception coil 87 and the power reception capacitor 89 have a predetermined series resonance frequency (e.g., 13.56 MHz) that coincides with the series resonance frequency of the power transmission coil units incorporated in the respective trocars explained above.

That is, on an inside of the bipolar electric knife 81, a power reception coil unit functioning as a series resonance circuit including the power reception coil 87 and the power reception capacitor 89 is provided.

Even when a medical wireless power supply system including the bipolar electric knife 81 including the configuration explained above and one of the respective trocars explained above is used, it is possible to perform power supply at relatively high transmission efficiency while making use of the magnetic field resonance phenomenon.

With the bipolar electric knife 81 including the configuration explained above, the power reception coil 87 can be increased in size (as illustrated in Fig. 11). Therefore, it is possible to easily improve transmission efficiency in power supply performed making use of the magnetic field resonance phenomenon.

As explained above, according to the present example, in a medical wireless power supply system including a trocar and a medical instrument such as a bipolar electric knife inserted into the trocar and used, it is possible to perform power supply at relatively high transmission efficiency while improving a degree of freedom of arrangement of a portion where a power transmission coil of the trocar is provided and a portion where a power reception coil of the medical instrument is provided. That is, with the medical wireless power supply system in the present example, it is possible to improve a degree of freedom of arrangement of portions related to power transmission and power reception while preventing deterioration in transmission efficiency of electric power as much as possible.

Note that, according to the present example, for example, a plurality of power transmission capacitors may be provided in the trocar. Further, a series resonance circuit may be formed between one power transmission capacitor selected out of the plurality of power transmission capacitors on the basis of operation of a not-shown switch or the like of the power supply device 21 and the power transmission coil provided in the trocar.

### (Second Example)

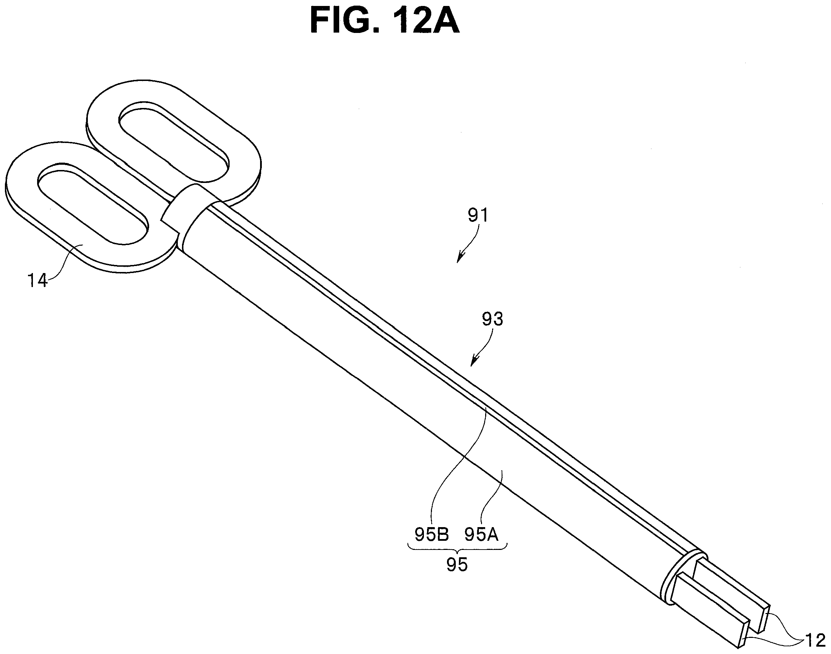

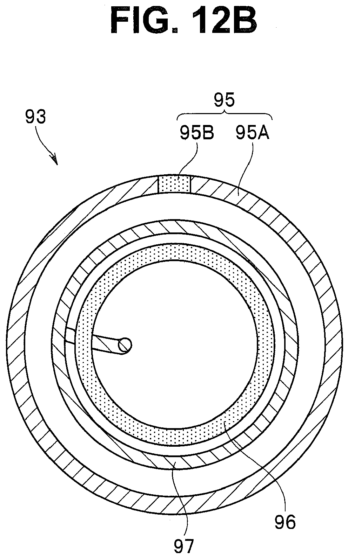

Fig. 12A to Fig. 17B relate to a second example. Note that, in the present example, detailed explanation concerning portions including configurations and the like same as those in the first example is omitted. Portions including configurations and the like different from those in the first example are mainly explained. Fig. 12A is a diagram showing an external appearance of a bipolar electric knife according to the second example. Fig. 12B is a sectional view showing a configuration of an insertion portion of the bipolar electric knife in Fig. 12A.

As shown in Fig. 12A, a bipolar electric knife 91 includes, at a distal end portion, the treatment electrodes 12 capable of applying a high-frequency current for performing treatment such as dissection or coagulation to a living tissue, includes, in a halfway portion, an insertion portion 93 having an elongated shape insertable into the insertion holes of the respective trocars explained in the first example, and includes, at a rear end portion, the operation portion 14 that enables operation related to opening and closing motions of the treatment electrodes 12 to be manually performed.

As shown in Fig. 12A and Fig. 12B, the bipolar electric knife 91 is configured such that water tightness of an inside of the insertion portion 93 is kept by an outer cylinder portion 95 including a substantially cylindrical tube portion 95A formed by a conductive member such as stainless steel and provided with a notch along a major axis direction and an insulating portion 95B formed by an insulating member such as resin and provided to fill a space of a notch portion of the tube portion 95A.

Further, as shown in Fig. 12B, on an inside of the outer cylinder portion 95, a cylindrical inner cylinder portion 96 formed by an insulating member such as resin and a power reception coil 97 wound along an outer circumference portion of the inner cylinder portion 96 are provided.

Note that, on the inside of the outer cylinder portion 95 in the present example, although not shown in the figure, a conversion circuit (same as the conversion circuit in the first example) capable of converting a waveform of an alternating current received in the power reception coil 97 into a waveform suitable for treatment (such as dissection or coagulation) and driving the treatment electrodes 12 and a power reception capacitor (same as the power reception capacitor in the first example) connected in series to the power reception coil 97 and the conversion circuit are provided.

The power reception coil 97 in the present example includes a winding state and a connection state same as the states of any one of the power reception coils 17, 77, and 87.

On the other hand, in the bipolar electric knife 91, inductance of the power reception coil 97 and capacitance of the not-shown power reception capacitor are respectively set such that the power reception coil 97 and the not-shown power reception capacitor have a predetermined series resonance frequency (e.g., 13.56 MHz) that coincides with the series resonance frequency of the power transmission coil units incorporated in the respective trocars explained in the first example

That is, on the inside of the bipolar electric knife 91, a power reception coil unit substantially the same as the power reception coil unit in the first example including the power reception coil 97 and the not-shown power reception capacitor is provided.

Even when a medical wireless power supply system including the bipolar electric knife 91 including the configuration explained above and one of the respective trocars explained in the first example is used, it is possible to perform power supply at relatively high transmission efficiency while making use of the magnetic field resonance phenomenon.

With the bipolar electric knife 91 including the configuration explained above, it is possible to perform wireless power supply making use of the magnetic field resonance phenomenon while forming the tube portion 95A of the outer cylinder portion 95 with a member having relatively high strength such as stainless steel.

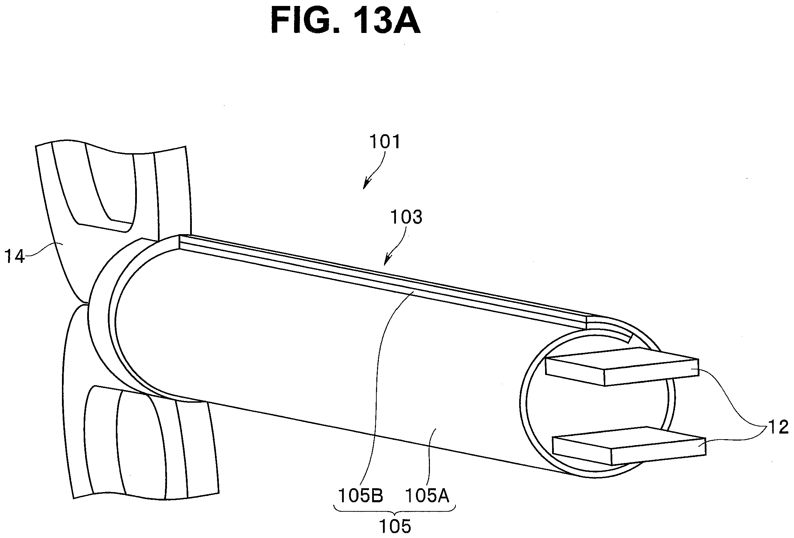

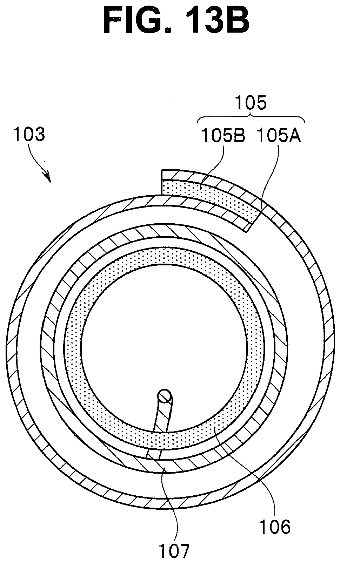

Note that, in the present example, instead of the bipolar electric knife 91 including the configuration shown in Fig. 12A and Fig. 12B, for example, a bipolar electric knife 101 including a configuration shown in Fig. 13A and Fig. 13B may be used. Fig. 13A is a diagram showing an external appearance of a bipolar electric knife according to a first modification of the second example.

Fig. 13B is a sectional view showing a configuration of an insertion portion of the bipolar electric knife in Fig. 13A.

As shown in Fig. 13A, the bipolar electric knife 101 includes, at a distal end portion, the treatment electrodes 12 capable of applying a high-frequency current for performing treatment such as dissection or coagulation to a living tissue, includes, in a halfway portion, an insertion portion 103 having an elongated shape insertable into the insertion holes of the respective trocars explained in the first example, and includes, at a rear end portion, the operation portion 14 that enables operation related to opening and closing motions of the treatment electrodes 12 to be manually performed.

As shown in Fig. 13A and Fig. 13B, the bipolar electric knife 101 is configured such that water tightness of an inside of the insertion portion 103 is kept by an outer cylinder portion 105 including a substantially cylindrical tube portion 105A formed by winding a conductive member such as stainless steel in a swirl shape (or a spiral shape) and an insulating portion 105B provided to fill a space formed between overlapping portions of the conductive member of the tube portion 105A.

Further, as shown in Fig. 13B, on an inside of the outer cylinder portion 105, a cylindrical inner cylinder portion 106 formed by an insulating member such as resin and a power reception coil 107 wound along an outer circumference portion of the inner cylinder portion 106 are provided.

Note that, on the inside of the outer cylinder portion 105 in the present example, although not shown in the figure, a conversion circuit (same as the conversion circuit in the first example) capable of converting a waveform of an alternating current received in the power reception coil 107 into a waveform suitable for treatment (such as dissection or coagulation) and driving the treatment electrodes 12 and a power reception capacitor (same as the power reception capacitor in the first example) connected in series to the power reception coil 107 and the conversion circuit are provided.

The power reception coil 107 in the present example includes a winding state and a connection state same as the states of any one of the power reception coils 17, 77, and 87.

On the other hand, in the bipolar electric knife 101, inductance of the power reception coil 107 and capacitance of the not-shown power reception capacitor are respectively set such that the power reception coil 107 and the not-shown power reception capacitor have a predetermined series resonance frequency (e.g., 13.56 MHz) that coincides with the series resonance frequency of the power transmission coil units incorporated in the respective trocars explained in the first example.

That is, on the inside of the bipolar electric knife 101, a power reception coil unit substantially the same as the power reception coil unit in the first example including the power reception coil 107 and the not-shown power reception capacitor is provided.

Even when a medical wireless power supply system including the bipolar electric knife 101 including the configuration explained above and one of the respective trocars explained in the first example is used, it is possible to perform power supply at relatively high transmission efficiency while making use of the magnetic field resonance phenomenon.

With the bipolar electric knife 101 including the configuration explained above, it is possible to perform wireless power supply making use of the magnetic field resonance phenomenon while increasing strength and water tightness of the outer cylinder portion 105.

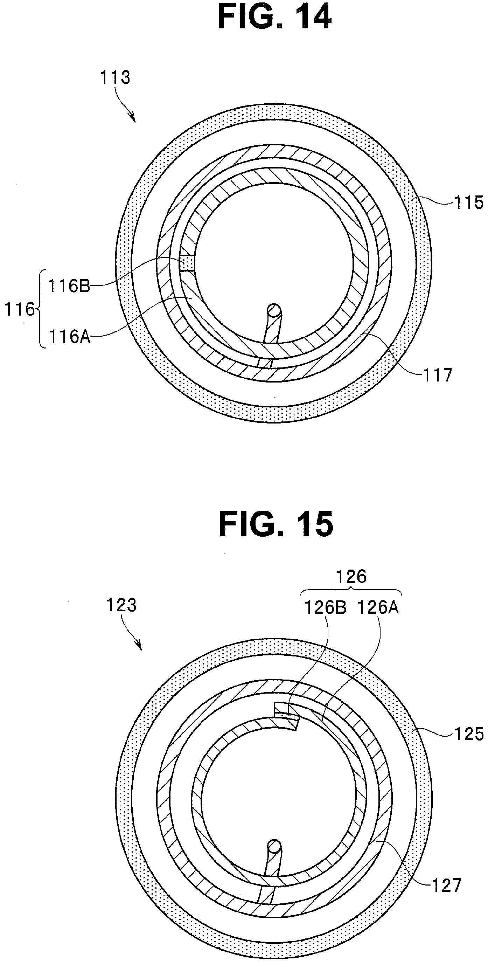

On the other hand, in the present example, instead of the insertion portion including the configuration shown in Fig. 12A to Fig. 13B, an insertion portion of a bipolar electric knife may be configured by an insertion portion 113 including a configuration shown in Fig. 14. Fig. 14 is a sectional view showing a configuration of an insertion portion according to a second modification of the second example

As shown in Fig. 14, the insertion portion 113 is configured such that water tightness on an inside is kept by a cylindrical outer cylinder portion 115 formed by an insulating member such as resin.

As shown in Fig. 14, on an inside of the outer cylinder portion 115, an inner cylinder portion 116 including a substantially cylindrical tube portion 116A formed by a conductive member such as stainless steel and provided with a notch along a major axis direction and an insulating portion 116B formed by an insulating member such as resin and provided to fill a space of a notch portion of the tube portion 116A is provided.

Further, as shown in Fig. 14, on the inside of the outer cylinder portion 115, a power reception coil 117 wound along an outer circumference portion of the inner cylinder portion 116 is provided.

Note that, on the inside of the outer cylinder portion 115 in the present example, although not shown in the figure, a conversion circuit (same as the conversion circuit in the first example) capable of converting a waveform of an alternating current received in the power reception coil 117 into a waveform suitable for treatment (such as dissection or coagulation) and driving the treatment electrodes 12 and a power reception capacitor (same as the power reception capacitor in the first example) connected in series to the power reception coil 117 and the conversion circuit are provided.

The power reception coil 117 in the present example includes a winding state and a connection state same as the states of any one of the power reception coils 17, 77, and 87.

On the other hand, inductance of the power reception coil 117 and capacitance of the not-shown power reception capacitor are respectively set such that the power reception coil 117 and the not-shown power reception capacitor have a predetermined series resonance frequency (e.g., 13.56 MHz) that coincides with the series resonance frequency of the power transmission coil units incorporated in the respective trocars explained in the first example.

That is, on the inside of the outer cylinder portion 115, a power reception coil unit substantially the same as the power reception coil unit in the first example including the power reception coil 117 and the not-shown power reception capacitor is provided.

In the present example, instead of the insertion portion including the configuration shown in Fig. 12A to Fig. 14, an insertion portion of a bipolar electric knife may be configured by an insertion portion 123 including a configuration shown in Fig. 15. Fig. 15 is a sectional view showing a configuration of an insertion portion according to a third modification of the second example.

As shown in Fig. 15, the insertion portion 123 is configured such that water tightness on an inside is kept by a cylindrical outer cylinder portion 125 formed by an insulating member such as resin.

As shown in Fig. 15, on an inside of the outer cylinder portion 125, an inner cylinder portion 126 including a substantially cylindrical tube portion 126A formed by winding a conductive member such as stainless steel in a swirl shape (or a spiral shape) and an insulating portion 126B provided to fill a space formed between overlapping portions of the conductive member of the tube portion 126A is provided.

Further, as shown in Fig. 15, on the inside of the outer cylinder portion 125, a power reception coil 127 wound along an outer circumference portion of the inner cylinder portion 126 is provided.

Note that, on the inside of the outer cylinder portion 125 in the present example, although not shown in the figure, a conversion circuit (same as the conversion circuit in the first example) capable of converting a waveform of an alternating current received in the power reception coil 127 into a waveform suitable for treatment (such as dissection or coagulation) and driving the treatment electrodes 12 and a power reception capacitor (same as the power reception capacitor in the first example) connected in series to the power reception coil 127 and the conversion circuit are provided.

The power reception coil 127 in the present example includes a winding state and a connection state same as the states of any one of the power reception coils 17, 77, and 87.

On the other hand, inductance of the power reception coil 127 and capacitance of the not-shown power reception capacitor are respectively set such that the power reception coil 127 and the not-shown power reception capacitor have a predetermined series resonance frequency (e.g., 13.56 MHz) that coincides with the series resonance frequency of the power transmission coil units incorporated in the respective trocars explained in the first example.

That is, on the inside of the outer cylinder portion 125, a power reception coil unit substantially the same as the power reception coil unit in the first example including the power reception coil 127 and the not-shown power reception capacitor is provided.

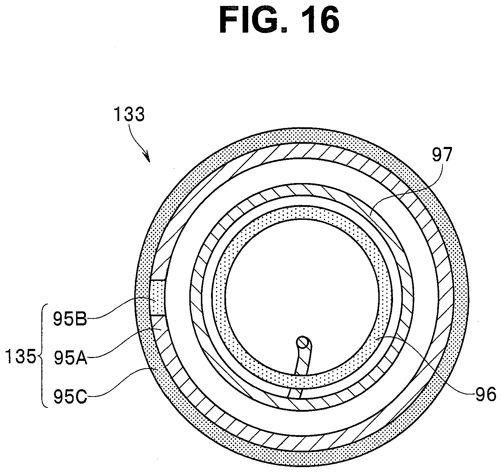

In the present example, for example, as shown as an insertion portion 133 in Fig. 16, a laminated outer cylinder portion 135 in which an insulating layer 95C formed by an insulating member such as resin is laminated to cover an outer circumferential surface of a layer including the tube portion 95A and the insulating portion 95B may be used instead of the outer cylinder portion 95 shown in Fig. 12A and Fig. 12B. Fig. 16 is a sectional view showing a configuration of an insertion portion according to a fourth modification of the second example.

With the insertion portion 133 including the laminated outer cylinder portion 135 explained above, it is possible to improve safety in performing wireless power supply making use of the magnetic field resonance phenomenon.

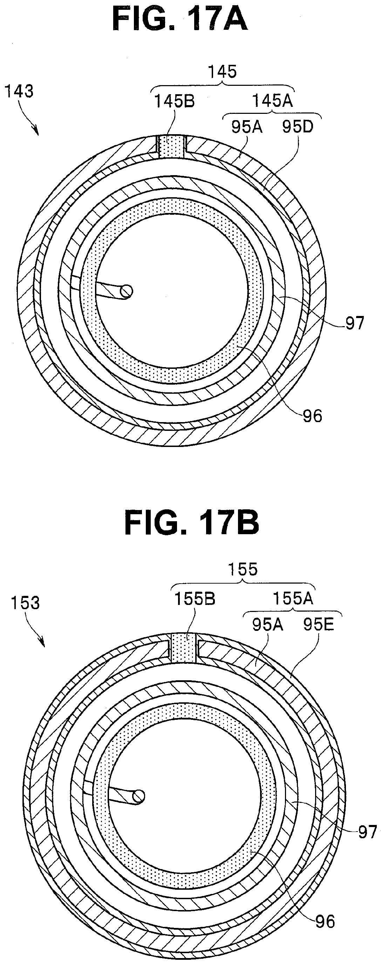

In the present example, for example, as shown as an insertion portion 143 in Fig. 17A, a laminated outer cylinder portion 145 including a laminated tube portion 145A and an insulating portion 145B may be used instead of the outer cylinder portion 95 shown in Fig. 12A and Fig. 12B. Fig. 17A is a sectional view showing a configuration of an insertion portion according to a fifth modification of the second example.

In the laminated tube portion 145A, a high conductive layer 95D formed of copper or the like having high conductivity compared with the tube portion 95A is laminated on an inner circumference of a layer of the tube portion 95A. The laminated tube portion 145A has a substantially cylindrical shape in which a notch is provided along a major axis direction.

The insulating portion 145B is formed by an insulating member such as resin and provided to fill a space in a notch portion of the laminated tube portion 145A.

With the insertion portion 143 including the laminated outer cylinder portion 145 explained above, it is possible to reduce a resistance loss at the time when an induction current flows to the laminated tube portion 145A because of a magnetic field applied from an outside.

In the present example, for example, as shown as an insertion portion 153 in Fig. 17B, a laminated outer cylinder portion 155 including a laminated tube portion 155A and an insulating portion 155B may be used instead of the outer cylinder portion 95 shown in Fig. 12A and Fig. 12B. Fig. 17B is a sectional view showing a configuration of an insertion portion according to a sixth modification of the second example.

In the laminated tube portion 155A, a high conductive layer 95E formed of copper or the like having high conductivity compared with the tube portion 95A is laminated to cover an inner circumferential surface and an outer circumferential surface of the layer of the tube portion 95A. The laminated tube portion 155A has a substantially cylindrical shape in which a notch is provided along a major axis direction.

The insulating portion 155B is formed by an insulating member such as resin and provided to fill a space in a notch portion of the laminated tube portion 155A.

With the insertion portion 153 including the laminated outer cylinder portion 155 explained above, it is possible to reduce a resistance loss at the time when an induction current flows to the laminated tube portion 155A because of a magnetic field applied from an outside.

Note that, according to the present example, the medical instrument such as the bipolar electric knife is not limited to the medical instrument including the insertion portion in which the insulating portion is provided only in one place of the outer cylinder and (or) the inner cylinder. The medical instrument may be, for example, a medical instrument including an insertion portion in which insulating portions are provided in a plurality of places in an outer cylinder portion and (or) an inner cylinder portion.

### (Third Example)

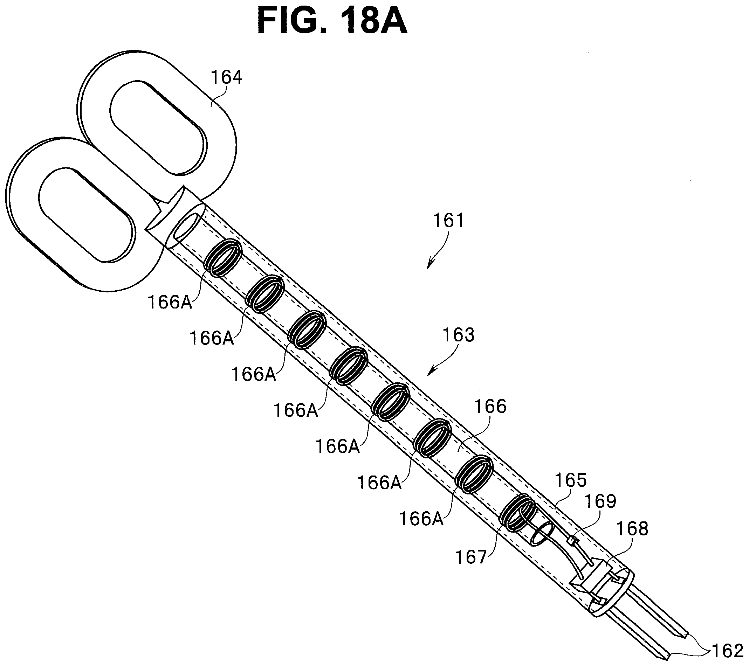

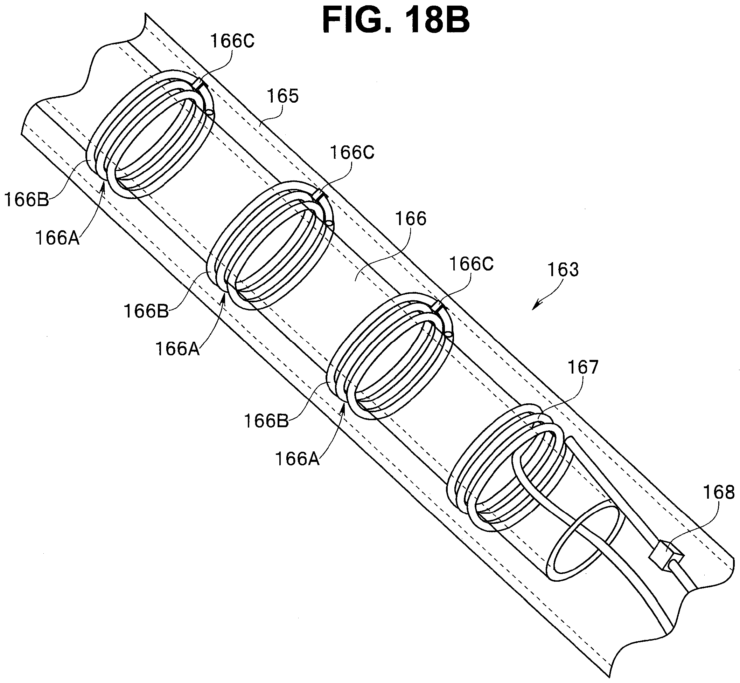

Fig. 18A and Fig. 18B relate to a third example of the present invention.

Note that, in the present example, detailed explanation concerning portions including configurations and the like same as those in one of the first and second examples is omitted. Portions including configurations and the like different from those in both of the first and second examples are mainly explained. Fig. 18A is a diagram showing a configuration of a main part of a bipolar electric knife according to the third example.

Fig. 18B is a sectional view showing a part of an insertion portion of the bipolar electric knife in Fig. 18A in enlargement.

As shown in Fig. 18A, a bipolar electric knife 161 includes, at a distal end portion, treatment electrodes 162 capable of applying a high-frequency current for performing treatment such as dissection or coagulation to a living tissue, includes, in a halfway portion, an insertion portion 163 having an elongated shape insertable into the insertion holes of the respective trocars explained above, and includes, at a rear end portion, an operation portion 164 that enables operation related to opening and closing motions of the treatment electrodes 162 to be manually performed.

As shown in Fig. 18A, the bipolar electric knife 161 is configured such that water tightness of an inside of the insertion portion 163 is kept by a cylindrical outer cylinder portion 165 formed by an insulating member such as resin.

Further, as shown in Fig. 18A, on an inside of the outer cylinder portion 165, a cylindrical inner cylinder portion 166 formed by an insulating member such as resin, a relay coil unit 166A and a power reception coil 167 wound along an outer circumference portion of the inner cylinder portion 166, a conversion circuit 168 capable of converting a waveform of an alternating current received in the power reception coil 167 into a waveform suitable for treatment (such as dissection or coagulation) and driving the treatment electrodes 162, and a power reception capacitor 169 connected in series to the power reception coil 167 and the conversion circuit 168 are provided.

As shown in Fig. 18A and Fig. 18B, one or a plurality of relay coil units 166A are provided on the inside of the outer cylinder portion 165 and in the outer circumference portion of the inner cylinder portion 166. Each of the relay coil units 166A includes one set of a relay coil 166B and a relay capacitor 166C connected in series. The relay coil 166B of the relay coil unit 166A is wound along the outer circumference portion of the inner cylinder portion 166 to include a winding axis parallel to a major axis of the insertion portion 163. (Note that, as shown in Fig. 18B, when the plurality of relay coil units 166A are provided, the relay coil units 166A are arranged to be spaced apart from one another.)

The power reception coil 167 is wound to include, on the inside of the outer cylinder portion 165 and in the outer circumference portion of the inner cylinder portion 166, a winding axis parallel to a major axis of the insertion portion 163 and generally cover a vicinity of the treatment electrodes 162. One end portion of the power reception coil 167 is connected to the power reception capacitor 169. The other end portion of the power reception coil 167 is connected to the conversion circuit 168.

On the other hand, in the bipolar electric knife 161, inductance of the power reception coil 167 and capacitance of the power reception capacitor 169 are respectively set such that the power reception coil 167 and the power reception capacitor 169 have a predetermined series resonance frequency (e.g., 13.56 MHz) that coincides with the series resonance frequency of the power transmission coil units incorporated in the respective trocars explained in the first example.

That is, on the inside of the bipolar electric knife 161, a power reception coil unit functioning as a series resonance circuit including the power reception coil 167 and the power reception capacitor 169 is provided.

In the bipolar electric knife 161, inductance of the relay coil 166B and capacitance of the relay capacitor 166C are respectively set such that the relay coil 166B and the relay capacitor 166C have a predetermined series resonance frequency (e.g., 13.56 MHz) that coincides with the series resonance frequency of the power transmission coil units incorporated in the respective trocars explained in the first example and the series resonance frequency of the power reception coil unit including the power reception coil 167 and the power reception capacitor 169.

That is, with the bipolar electric knife 161, when power transmission is performed from any one of the trocars explained in the first example, it is possible to transmit electric power to the power reception coil unit including the power reception coil 167 and the power reception capacitor 169 via any relay coil unit 166A.

Even when a medical wireless power supply system including the bipolar electric knife 161 including the configuration explained above and one of the respective trocars explained in the first example is used, it is possible to perform power supply at relatively high transmission efficiency while making use of the magnetic field resonance phenomenon.

With the bipolar electric knife 161 including the configuration explained above, the relay coil 166B and the power reception coil 167 can be reduced in size. Therefore, it is possible to improve assembly accuracy in manufacturing of the insertion portion 163.

Note that, according to the present example, for example, by applying any one of the respective configurations explained in the second example to the bipolar electric knife 161, it is possible to attain improvement of strength of the insertion portion 163 and (or) improvement of transmission efficiency in power supply.

### (Fourth Example)

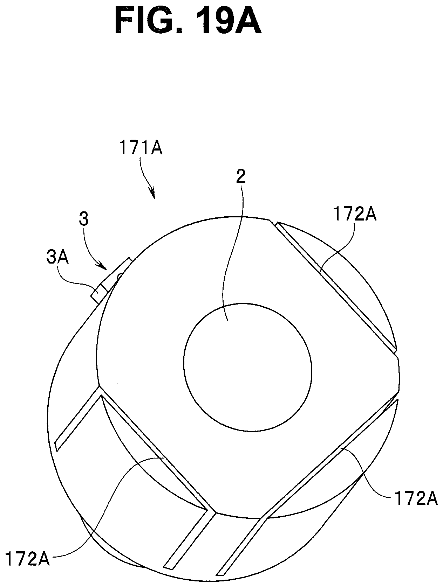

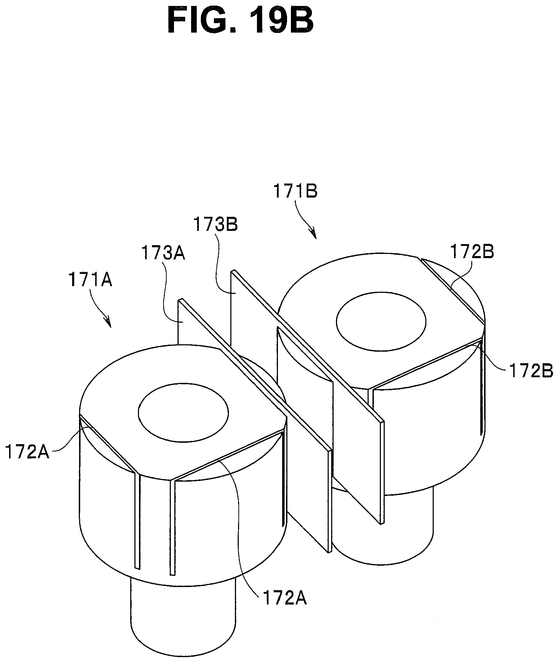

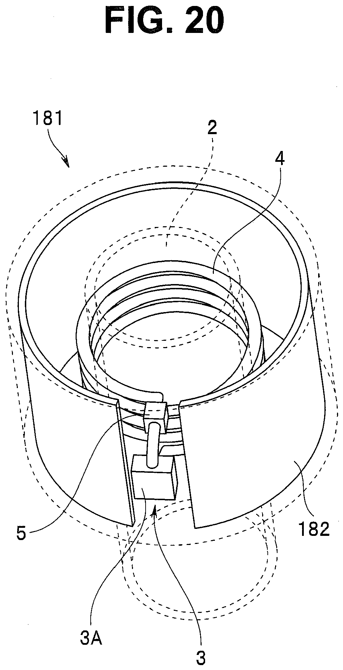

Fig. 19A, Fig. 19B, and Fig. 20 relate to a fourth example.

Note that, in the present example, detailed explanation concerning portions including configurations and the like same as those in any one of the first to third examples is omitted. Portions including configurations and the like different from those in all of the first to third examples are mainly explained.
Fig. 19A is a diagram showing a configuration of a trocar according to the fourth example.

Fig. 19B is a diagram showing an example of an aspect of use of the trocar shown in Fig 19A.

As shown in Fig. 19A, a trocar 171A is configured by providing a plurality of notch portions 172A around the insertion hole 2 of an armor portion of the trocar 1 explained in the first example.

Conductive plates 173A explained below are detachably attachable to the plurality of notch portions 172A. The plurality of notch portions 172A are formed to respectively have shapes capable of holding the attached conductive plates 173A.

On the other hand, for example, as shown in Fig. 19B, the trocar 171A in the present example is configured assuming that the trocar 171A is used simultaneously with a trocar 171B, a resonance frequency of a power transmission coil unit of which is set the same as a resonance frequency of a power transmission coil unit of the trocar 171A.

More specifically, as shown in Fig. 19B, when being used simultaneously with the trocar 171B, the trocar 171A is used in a state in which the conductive plate 173A is attached to the notch portion 172A closest to the trocar 171B. As shown in Fig. 19B, when being used simultaneously with the trocar 171A, the trocar 171B is used in a state in which a conductive plate 173B is attached to a notch portion 172B closest to the trocar 171A.

The conductive plate 173A is formed as a tabular partition member having sufficiently large width and height with respect to a diameter and height of a power transmission coil included in the power transmission coil unit incorporated in the trocar 171A. The conductive plate 173B is configured as a tabular member having sufficiently large width and height with respect to a diameter and height of a power transmission coil included in the power transmission coil unit incorporated in the trocar 171B.

The trocar 171A is used in a state in which the conductive plate 173A explained above is set, whereby it is possible to suppress a leak of a magnetic field to the trocar 171B side. The trocar 171B is used in a state in which the conductive plate 173B is set, whereby it is possible to suppress a leak of a magnetic field to the trocar 171A side.

Note that, in the present example, the trocar is not limited to the trocar including the configuration like the trocars 171A and 171B and may include, for example, a configuration like a trocar 181 in Fig. 20. Fig. 20 is a diagram showing a configuration of a trocar according to a modification of the fourth example.

As shown in Fig. 20, the trocar 181 is configured by providing a conductive plate 182 around the power transmission coil 4 on the inside of the trocar 1 explained in the first example.

As shown in Fig. 20, the conductive plate 182 is formed as a partition member having sufficiently large height with respect to height of the power transmission coil 4 incorporated in the trocar 181 and capable of generally covering the outer circumference portion of the power transmission coil 4 in positions respectively not in contact with the respective portions of the power transmission coil 4 and the power transmission capacitor 5.

Note that the conductive plate 182 may be formed in a C shape including a notch shown in Fig. 20 or may be formed to be provided with the insulating member shown in Fig. 12A and Fig. 12B as long as the conductive plate 182 includes an insulating portion in a part of an outer circumference thereof.

Note that, in the trocar 181, not-shown insulating members are provided around the electric terminal 3A and the power transmission capacitor 5, whereby the electric terminal 3A and the power transmission capacitor 5 are electrically insulated from the conductive plate 182.

According to the configuration of the present example explained above, even when resonance frequencies of power transmission coil units respectively provided in a plurality of trocars are set the same as one another, it is possible to simultaneously perform power supply to the plurality of trocars.

### (fifth Example)

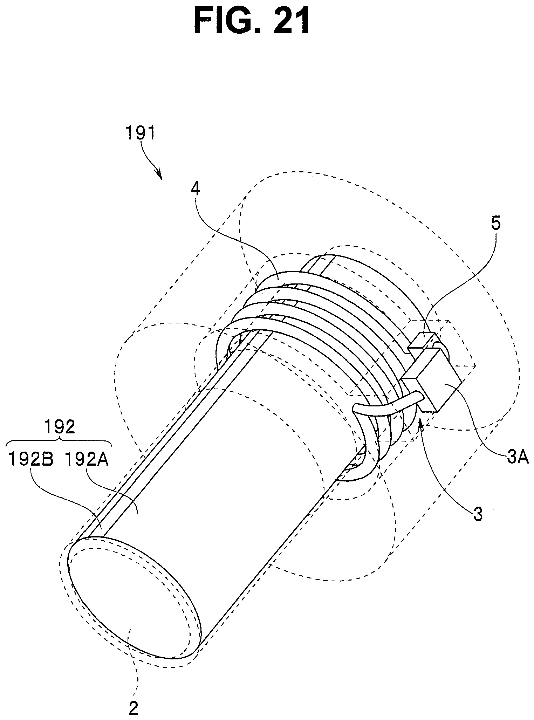

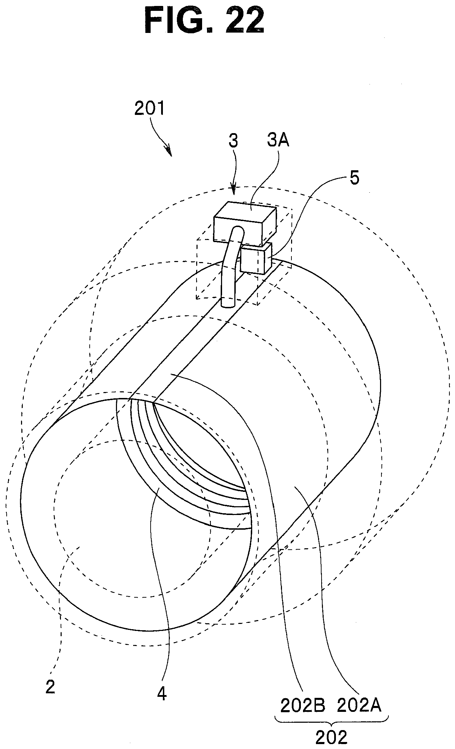

Fig. 21 and Fig. 22 relate to a fifth example.

Note that, in the present example, detailed explanation concerning portions including configurations and the like same as those in any one of the first to fourth examples is omitted. Portions including configurations and the like different from those in all of the first to fourth examples are mainly explained. Fig. 21 is a diagram showing a configuration of a trocar according to the fifth example.

As shown in Fig. 21, a trocar 191 is configured by providing an inner cylinder portion 192 around the insertion hole 2 on the inside of the trocar 1 and on the inner side of the power transmission coil 4 explained in the first example.

As shown in Fig. 21, the inner cylinder portion 192 is provided in a portion ranging from a vicinity of the power transmission coil 4 to an opening portion on an outlet side of the insertion hole 2. The inner cylinder portion 192 includes a substantially cylindrical tube portion 192A formed by a conductive member such as copper and provided with a notch along a major axis direction and an insulating portion 192B formed by an insulating member such as resin and provided to fill a space of a notch portion of the tube portion 192A.

Note that the insulating portion 192B is not limited to an insulating portion formed by the insulating member such as resin and may be a space itself of the notch portion of the tube portion 192A.

With the trocar 191 including the configuration explained above, it is easy to improve transmission efficiency in power supply performed making use of the magnetic field resonance phenomenon.

Note that, in the present example, the trocar is not limited to the trocar including the configuration like the trocar 191 and may include, for example, a configuration like a trocar 201 in Fig. 22. Fig. 22 is a diagram showing a configuration of a trocar according to a modification of the fifth example.

As shown in Fig. 22, the trocar 201 is configured by providing an outer cylinder portion 202 around the insertion hole 2 on the inside of the trocar 1 and on the outer side of the power transmission coil 4 explained in the first example.

As shown in Fig. 22, the outer cylinder portion 202 is provided in a portion ranging from the vicinity of the power transmission coil 4 to the opening portion on the outlet side of the insertion hole 2. The outer cylinder portion 202 includes a substantially cylindrical tube portion 202A formed by a conductive member such as copper and provided with a notch along a major axis direction and an insulating portion 202B formed by an insulating member such as resin and provided to fill a space of a notch portion of the tube portion 202A.

Note that the insulating portion 202B is not limited to the insulating portion formed by the insulating member such as resin and may be a space itself of the notch portion of the tube portion 202A.

In the trocar 201, a part of a wire in connecting the electric terminal 3A, the power transmission coil 4, and the power transmission capacitor 5 in series is provided to pierce through the insulating portion 202B and not to be in contact with the tube portion 202A.

With the trocar 201 including the configuration explained above, it is easy to improve transmission efficiency in power supply performed making use of the magnetic field resonance phenomenon.

Note that, in the present example, for example, the inner cylinder portion 192 may be formed to include a configuration substantially the same as the configuration of the inner cylinder portion 126 in the second example.

In the present example, for example, the outer cylinder portion 202 may be formed to include a configuration substantially the same as the configuration of the outer cylinder portion 105 in the second embodiment.

On the other hand, the inner cylinder portion 192 in the present example may include a plurality of insulating portions 192B. The outer cylinder portion 202 in the present example may include a plurality of insulating portions 202B.

### (Sixth Example)

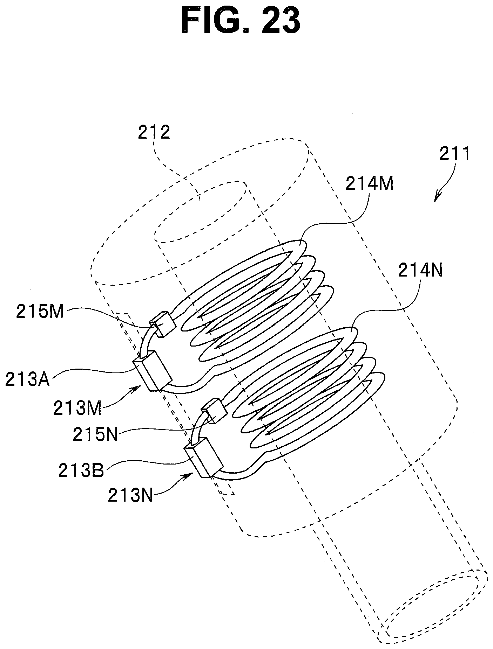

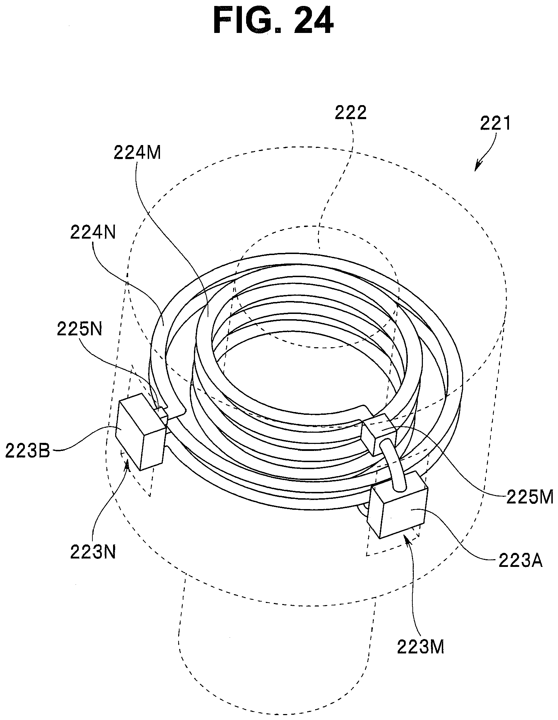

Fig. 23 and Fig. 24 relate to a sixth example, which is an embodiment of the present invention.

Note that, in the present example, detailed explanation concerning portions including configurations and the like same as those in any one of the first to fifth examples is omitted. Portions including configurations and the like different from those in all of the first to fifth examples are mainly explained.
Fig. 23 is a diagram showing a configuration of a trocar according to the sixth example.

As shown in Fig. 23, a trocar 211 includes an insertion hole 212 formed as a hole having a dimension for enabling a treatment instrument and the like to be inserted and connection terminal portions 213M and 213N respectively formed such that the power transmission cable 24 can be detachably connected thereto.

As shown in Fig. 23, on an inside of the trocar 211, a power transmission coil 214M wound along an outer circumference portion of the insertion hole 212 and a power transmission capacitor 215M connected in series to an electric terminal 213A provided in the connection terminal portion 213M and the power transmission coil 214M are provided.

Peripheries of the power transmission coil 214M and the power transmission capacitor 215M on the inside of the trocar 211 are covered with insulating members such as resin.

The power transmission coil 214M is wound to include, on the inside of the trocar 211 and in the outer circumference portion of the insertion hole 212, a winding axis parallel to an insertion axis of the insertion hole 212 and generally cover a portion close to an opening portion on an inlet side of the insertion hole 212 compared with a power transmission coil 214N explained below. One end portion of the power transmission coil 214M is connected to the power transmission capacitor 215M. The other end portion of the power transmission coil 214M is connected to the electric terminal 213A.

Further, in the trocar 211, inductance of the power transmission coil 214M and capacitance of the power transmission capacitor 215M are respectively set such that the power transmission coil 214M and the power transmission capacitor 215M have a predetermined series resonance frequency (e.g., 13.56 MHz) that coincides with the series resonance frequency of the power reception coil units incorporated in the respective bipolar electric knives explained in the second example and the like.

On the other hand, as shown in Fig. 23, on the inside of the trocar 211, a power transmission coil 214N wound along the outer circumference portion of the insertion hole 212 and a power transmission capacitor 215N connected in series to an electric terminal 213B provided in the connection terminal portion 213N and the power transmission coil 214N are provided.

Peripheries of the power transmission coil 214N and the power transmission capacitor 215N on the inside of the trocar 211 are covered with insulating members such as resin.

The power transmission coil 214N is wound to include, on the inside of the trocar 211 and in the outer circumference portion of the insertion hole 212, a winding axis parallel to the insertion axis of the insertion hole 212 and generally cover a portion away from the opening portion on the inlet side of the insertion hole 212 compared with the power transmission coil 214M. One end portion of the power transmission coil 214N is connected to the power transmission capacitor 215N. The other end portion of the power transmission coil 214N is connected to the electric terminal 213B.

Further, in the trocar 211, inductance of the power transmission coil 214N and capacitance of the power transmission capacitor 215N are respectively set such that the power transmission coil 214N and the power transmission capacitor 215N have a series resonance frequency (e.g., 10 MHz) different from the series resonance frequency set by the power transmission coil 214M and the power transmission capacitor 215M.

That is, on the inside of the trocar 211, two power transmission coil units, i.e., a power transmission coil unit including the power transmission coil 214M and the power transmission capacitor 215M and a power transmission coil unit including the power transmission coil 214N and the power transmission capacitor 215N are provided along the insertion axis of the insertion portion 212.

With the configuration of the trocar 211 explained above, for example, a medical instrument that performs power supply making use of the magnetic field resonance phenomenon can be used while being selected out of two medical instruments in which series resonance frequencies of power reception coil units are set to be different from each other.

Note that, with the configuration of the trocar 211 explained above, the connection terminal portions 213M and 213N may be arranged in positions close to each other as long as the electric terminals 213A and 213B are electrically separated from each other.

On the other hand, in the present example, the trocar is not limited to the trocar including the configuration like the trocar 211 and may include, for example, a configuration like a trocar 221 in Fig. 24. Fig. 24 is a diagram showing a configuration of a trocar according to a modification of the sixth example, which is an embodiment of the present invention.

As shown in Fig. 24, the trocar 221 includes an insertion hole 222 formed as a hole having a dimension for enabling a treatment instrument and the like to be inserted and connection terminal portions 223M and 223N respectively formed such that the power transmission cable 24 can be detachably connected thereto.

As shown in Fig. 24, on an inside of a trocar 221, a power transmission coil 224M wound along an outer circumference portion of the insertion hole 222 and a power transmission capacitor 225M connected in series to an electric terminal 223A provided in the connection terminal portion 223M and the power transmission coil 224M.

Peripheries of the power transmission coil 224M and the power transmission capacitor 225M on the inside of the trocar 221 are covered with insulating members such as resin.

The power transmission coil 224M is wound to include, on the inside of the trocar 221 and in the outer circumference portion of the insertion hole 222, a winding axis parallel to an insertion axis of the insertion hole 222 and generally cover a part of the insertion hole 222. One end portion of the power transmission coil 224M is connected to the power transmission capacitor 225M. The other end portion of the power transmission coil 224M is connected to the electric terminal 223A.

Further, in the trocar 221, inductance of the power transmission coil 224M and capacitance of the power transmission capacitor 225M are respectively set such that the power transmission coil 224M and the power transmission capacitor 225M have a predetermined series resonance frequency (e.g., 13.56 MHz) that coincides with a series resonance frequency of the power reception coil units incorporated in the respective bipolar electric knives explained in the second example and the like.

On the other hand, as shown in Fig. 24, on the inside of the trocar 221, a power transmission coil 224N wound along the outer circumference portion of the insertion hole 222 and a power transmission capacitor 225N connected in series to an electric terminal 223B provided in the connection terminal portion 223N and the power transmission coil 224N are provided.

Peripheries of the power transmission coil 224N and the power transmission capacitor 225N on the inside of the trocar 221 are covered with insulating members such as resin.

The power transmission coil 224N is wound to include, on the inside of the trocar 221 and in the outer circumference portion of the power transmission coil 224M, a winding axis parallel to the insertion axis of the insertion hole 222. One end portion of the power transmission coil 224N is connected to the power transmission capacitor 225N. The other end portion of the power transmission coil 224N is connected to the electric terminal 223B.

Further, in the trocar 221, inductance of the power transmission coil 224N and capacitance of the power transmission capacitor 225N are respectively set such that the power transmission coil 224N and the power transmission capacitor 225N have a series resonance frequency (e.g., 10 MHz) different from the series resonance frequency set by the power transmission coil 224M and the power transmission capacitor 225M.

That is, on the inside of the trocar 221, two power transmission coil units, i.e., a power transmission coil unit including the power transmission coil 224M and the power transmission capacitor 225M and a power transmission coil unit including the power transmission coil 224N and the power transmission capacitor 225N are provided to doubly surround a part of the outer circumference portion of the insertion hole 222.

With the configuration of the trocar 221 explained above, for example, a medical instrument that performs power supply making use of the magnetic field resonance phenomenon can be used while being selected out of two medical instruments in which series resonance frequencies of power reception coil units are set to be different from each other.

Note that, with the configuration of the trocar 221 explained above, the connection terminal portions 223M and 223N may be arranged in positions close to each other as long as the electric terminals 223A and 223B are electrically separated from each other.

On the other hand, the trocars 211 and 221 are not limited to the trocar including the configuration explained above. For example, by applying the aspect shown in Fig. 6A and Fig. 6B, the trocars 211 and 221 may be configured by providing one power transmission coil unit in the trocar main body portion 32A and providing the other power transmission coil unit in the power transmission coil unit 32B.

### (Seventh Example)

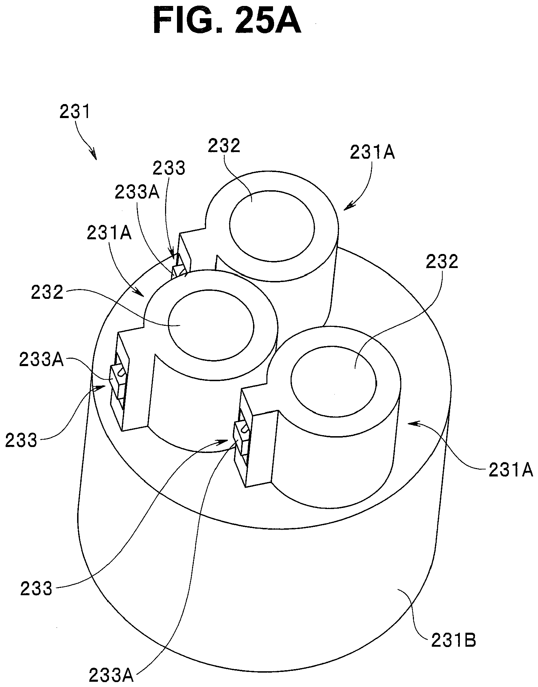

Fig. 25A to Fig. 27B relate to a seventh example, which is an embodiment of the present invention.

Note that, in the present example, detailed explanation concerning portions including configurations and the like same as those in any one of the first to sixth examples is omitted. Portions including configurations and the like different from those in all of the first to sixth examples are mainly explained.
Fig. 25A is a diagram showing an external appearance of a trocar according to the seventh example.

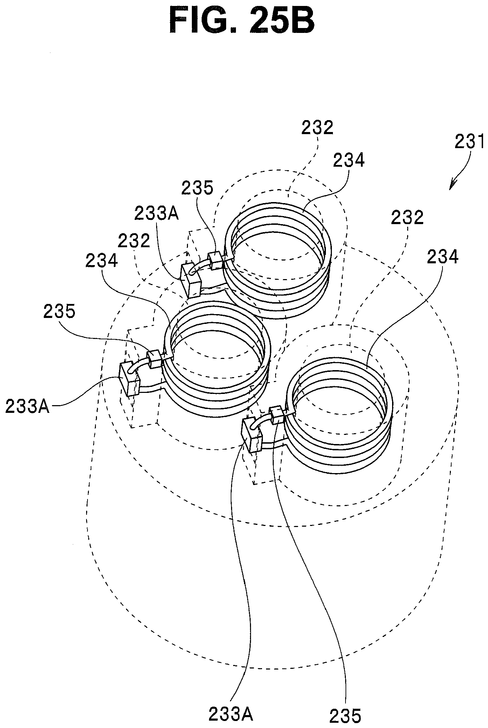

Fig. 25B is a diagram showing an internal configuration of a main part of the trocar according to the seventh example.

As shown in Fig. 25A, a trocar 231 is configured to make it possible to simultaneously insert a plurality of medical instruments from one incised wound formed on a body wall of a subject and use the medical instruments.

More specifically, as shown in Fig. 25A, the trocar 231 includes three insertion hole portions 231A and a cylinder portion 231B formed to communicate with each of opening portions (not shown in the figure) on outlet sides of insertion holes 232 provided one each in each of the insertion hole portions 231A.

As shown in Fig. 25A, in the respective insertion hole portions 231A, the insertion holes 232 formed as holes having a dimension for enabling a treatment instrument and the like to be inserted and connection terminal portions 233 formed such that the power transmission cable 24 can be detachably connected thereto are provided.

On the other hand, as shown in Fig. 25B, on insides of the respective insertion hole portions 231A, power transmission coils 234 wound along outer circumference portions of the insertion holes 232 and power transmission capacitors 235 connected in series to electric terminals 233A provided in the connection terminal portions 233 and the power transmission coils 234 are provided.

Peripheries of the power transmission coils 234 and the power transmission capacitors 235 on the insides of the respective insertion hole portions 231A are covered with insulating members such as resin.

The power transmission coils 234 are wound to include, on the insides of the insertion hole portions 231A and the outer circumference portions of the insertion holes 232, winding axes parallel to insertion axes of the insertion holes 232. One end portions of the power transmission coils 234 are connected to the power transmission capacitors 235. The other end portions of the power transmission coils 234 are connected to the electric terminals 233A.

Further, in the trocar 231, inductance of the power transmission coils 234 and capacitance of the power transmission capacitors 235 are respectively set such that the power transmission coils 234 and the power transmission capacitors 235 have a different series resonance frequency for each of the insertion hole portions 231A. More specifically, for example, in a first insertion hole portion 231A among the three insertion hole portions 231A, inductance of the power transmission coil 234 and capacitance of the power transmission capacitor 235 are set such that the power transmission coil 234 and the power transmission capacitor 235 have a first resonance frequency (e.g., 13.56 MHz). In a second insertion hole portion 231A, inductance of the power transmission coil 234 and capacitance of the power transmission capacitor 235 are set such that the power transmission coil 234 and the power transmission capacitor 235 have a second resonance frequency (e.g., 10 MHz). In a third insertion hole portion 231A, inductance of the power transmission coil 234 and capacitance of the power transmission capacitor 235 are set such that the power transmission coil 234 and the power transmission capacitor 235 have a third resonance frequency (e.g., 15 MHz).

That is, on an inside of the first insertion hole portion 231A, a first power transmission coil unit configured to resonate at the first resonance frequency is provided. On an inside of the second insertion hole portion 231A, a second power transmission coil unit configured to resonate at the second resonance frequency is provided. Further, on an inside of the third insertion hole portion 231A, a third power transmission coil unit configured to resonate at the third resonance frequency is provided.

With the configuration of the trocar 231 explained above, it is possible to simultaneously perform power supply due to the magnetic field resonance phenomenon to a plurality of medical instruments.

Note that, with the configuration of the trocar 231 explained above, the insertion hole portions 231A may be arranged in positions where the respective electric terminals 233A are close to one another.

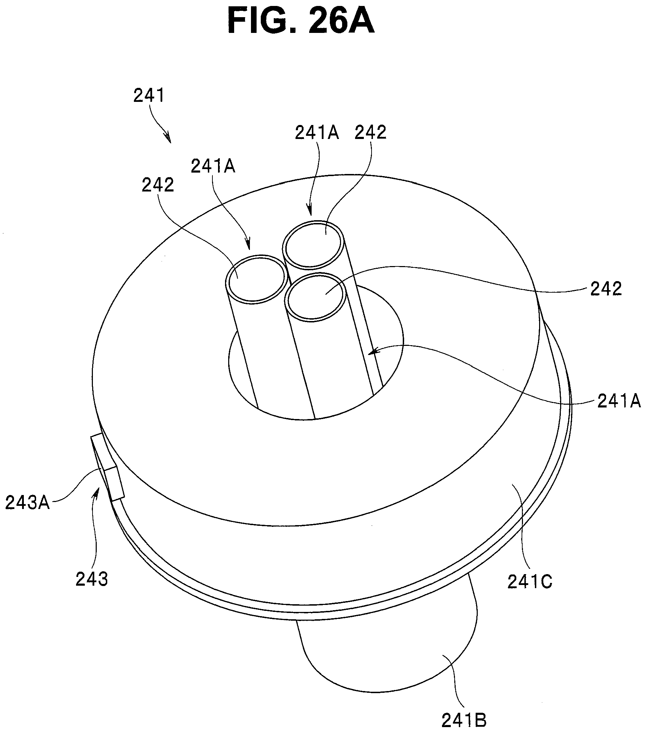

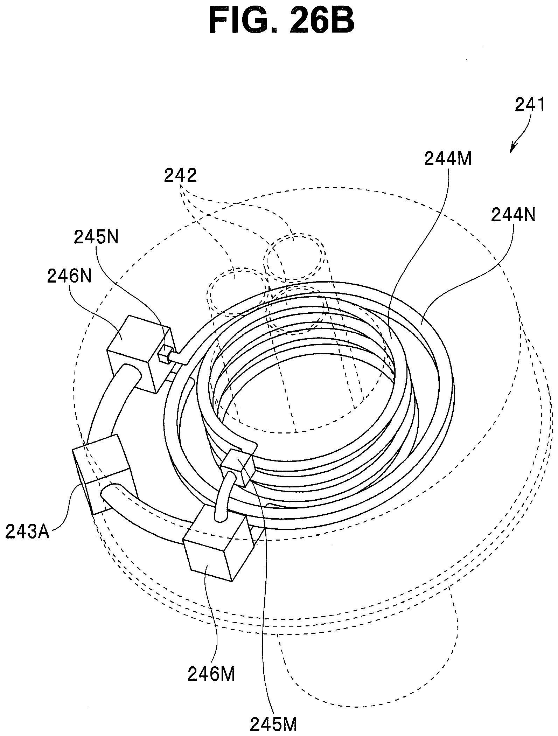

On the other hand, in the present example, the trocar is not limited to the trocar including the configuration like the trocar 231 and may include, for example, a configuration like a trocar 241 in Fig. 26A and Fig. 26B. Fig. 26A is a diagram showing an external appearance of a trocar according to a first modification of the seventh example, which is an embodiment of the present invention.

Fig. 26B is a diagram showing an internal configuration of a main part of the trocar according to the first modification of the seventh example

As shown in Fig. 26A, the trocar 241 is configured to make it possible to simultaneously insert a plurality of medical instruments from one incised wound formed on a body wall of a subject and use the medical instruments.

More specifically, as shown in Fig. 26A, the trocar 241 includes three insertion hole portions 241A, a cylinder portion 241B formed to communicate with each of opening portions (not shown in the figure) on outlet sides of insertion holes 242 provided one each in each of the insertion hole portions 241A, and a ring portion 241C formed to annularly surround outer circumferential sides of the respective insertion hole portions 241 A.

As shown in Fig. 26A, in the respective insertion hole portions 241A, the insertion holes 242 formed as holes having a dimension for enabling a treatment instrument and the like to be inserted are provided.

Further, as shown in Fig. 26A, a connection terminal portion 243 formed such that the power transmission cable 24 can be detachably connected thereto is provided on a side surface of the ring portion 241 C.

On the other hand, as shown in Fig. 26B, on an inside of the ring portion 241 C, a power transmission coil 244M wound to cover parts of outer circumference portions of the respective insertion holes 232, an electric connection member 246M connected to an electric terminal 243A of the connection terminal portion 243, and a power transmission capacitor 245M connected in series to the electric connection member 246M and the power transmission coil 244M are provided.

Peripheries of the power transmission coil 244M and the power transmission capacitor 245M on the inside of the ring portion 241C are covered with insulating members such as resin.

The power transmission coil 244M is wound to include, on the inside of the ring portion 241C and in the outer circumference portions of the respective insertion holes 222, a winding axis parallel to insertion axes of the respective insertion holes 242. One end portion of the power transmission coil 244M is connected to the power transmission capacitor 245M. The other end portion of the power transmission coil 244M is connected to the electric connection member 246M.

Further, in the trocar 241, inductance of the power transmission coil 244M and capacitance of the power transmission capacitor 245M are respectively set such that the power transmission coil 244M and the power transmission capacitor 245M have a predetermined series resonance frequency (e.g., 13.56 MHz) that coincides with the series resonance frequency of the power reception coil units incorporated in the respective bipolar electric knives explained in the second example and the like.

As shown in Fig. 26B, on the inside of the ring portion 241C, a power transmission coil 244N wound to cover parts of the outer circumference portions of the respective insertion holes 232, an electric connection member 246N connected to the electric terminal 243A of the connection terminal portion 243, and a power transmission capacitor 245N connected in series to the electric connection member 246N and the power transmission coil 244N are provided.

Further, peripheries of the power transmission coil 244N and the power transmission capacitor 245N on the inside of the ring portion 241C are covered with insulating members such as resin.

The power transmission coil 244N is wound to include, on the inside of the ring portion 241C and in an outer circumference portion of the power transmission coil 244M, a winding axis parallel to the insertion axes of the respective insertion holes 242. One end portion of the power transmission coil 244N is connected to the power transmission capacitor 245N. The other end portion of the power transmission coil 244N is connected to the electric connection member 246N.

Further, in the ring portion 241C, inductance of the power transmission coil 244N and capacitance of the power transmission capacitor 245N are respectively set such that the power transmission coil 244N and the power transmission capacitor 245N have a series resonance frequency (e.g., 10 MHz) different from the series resonance frequencies set by the power transmission coil 244M and the power transmission capacitor 245M.

That is, on the inside of the ring portion 241C, two power transmission coil units, i.e., a power transmission coil unit including the power transmission coil 244M and the power transmission capacitor 245M and a power transmission coil unit including the power transmission coil 244N and the power transmission capacitor 245N are provided to doubly surround a part of the outer circumference portions of the respective insertion holes 242.

With the configuration of the trocar 241 explained above, it is possible to simultaneously perform power supply due to the magnetic field resonance phenomenon to a plurality of medical instruments.

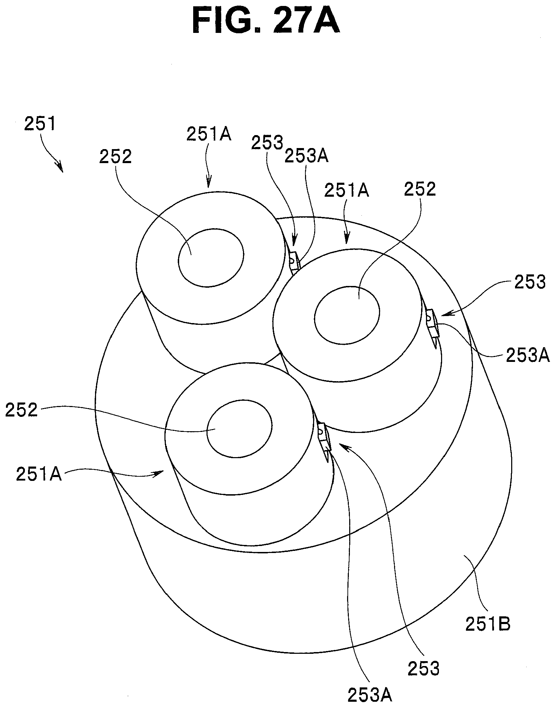

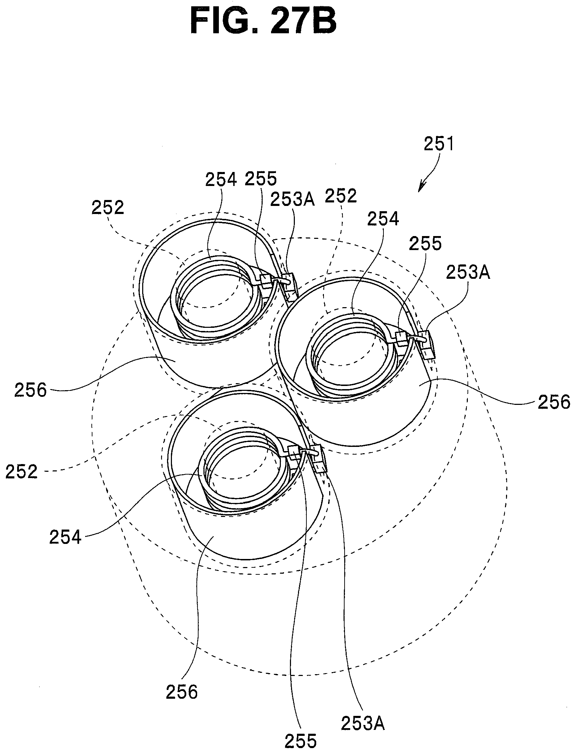

In the present example, the trocar is not limited to the trocar including the configuration like the trocar 231 or 241 and may include, for example, a configuration like a trocar 251 in Fig. 27A and Fig. 27B. Fig. 27A is a diagram showing an external appearance of a trocar according to a second modification of the seventh example, which however is not an embodiment of the invention.

Fig. 27B is a diagram showing an internal configuration of a main part of the trocar according to the second modification of the seventh example.

As shown in Fig. 27A, the trocar 251 is configured to make it possible to simultaneously insert a plurality of medical instruments from one incised wound formed on a body wall of a subject and use the medical instruments.

More specifically, as shown in Fig. 27A, the trocar 251 includes three insertion hole portions 251A and a cylinder portion 251B formed to communicate with each of opening portions (not shown in the figure) on outlet sides of the respective insertion holes 252 provided one each in each of the insertion hole portions 251A.

As shown in Fig. 27A, in the respective insertion hole portions 251A, insertion holes 252 formed as holes having a dimension for enabling a treatment instrument and the like to be inserted and connection terminal portions 253 formed such that the power transmission cable 24 can be detachably connected thereto are provided.

On the other hand, as shown in Fig. 27B, on insides of the respective insertion hole portions 251A, power transmission coils 254 wound along outer circumference portions of the insertion holes 252, power transmission capacitors 255 connected in series to electric terminals 253A provided in the connection terminal portions 253 and the power transmission coils 254, and conductive plates 256 formed as C-shaped tabular members capable of generally covering outer circumference portions of the power transmission coils 254 are provided.

Peripheries of the power transmission coils 254 and the power transmission capacitors 255 on the insides of the respective insertion hole portions 251A are covered with insulating members such as resin.

The power transmission coils 254 are wound to include, on the insides of the insertion hole portions 251A and the outer circumference portions of the insertion holes 252, winding axes parallel to insertion axes of the insertion holes 252. One end portions of the power transmission coils 254 are connected to the power transmission capacitors 255. The other end portions of the power transmission coils 254 are connected to the electric terminals 253A.

The conductive plates 256 are formed as partition members having sufficiently large height with respect to height of the power transmission coils 254 and capable of generally covering the outer circumference portions of the power transmission coils 254 in positions respectively not in contact with the respective portions of the electric terminals 253A, the power transmission coils 254 and the power transmission capacitors 255.

Note that the conductive plates 256 may be formed in a C shape including a notch shown in Fig. 27B as long as parts of outer circumferences of the conductive plates 256 include insulating portions. Alternatively, the conductive plates 256 may be formed with the insulating member shown in Fig. 12A and Fig. 12B provided therein.

Further, in the trocar 251, inductance of the power transmission coils 234 and capacitance of the power transmission capacitors 235 can be respectively set such that the respective insertion hole portions 251A have a same series resonance frequency. More specifically, for example, in the respective insertion hole portions 251A, inductance of the power transmission coils 254 and capacitance of the power transmission capacitors 255 can be respectively set such that the power transmission coils 254 and the power transmission capacitors 255 have a predetermined resonance frequency (e.g., 13.56 MHz).

That is, in the trocar 251, power transmission coil units configured to resonate at a predetermined resonance frequency can be provided on the insides of the respective insertion hole portions 251A.

With the configuration of the trocar 251 explained above, even when resonance frequencies of the power transmission coil units respectively provided in the respective insertion hole portions 251A are set the same, it is possible to simultaneously perform power supply due to the magnetic field resonance phenomenon to a plurality of medical instruments.

Note that, with the configuration of the trocar 251 explained above, for example, it is also possible to set the power transmission coil units of two insertion hole portions 251A among the respective insertion hole portions 251A to a first resonance frequency and set a resonance frequency of the power transmission coil unit of the remaining one insertion hole portion 251A to a second resonance frequency different from the first resonance frequency.

With the configuration of the trocar 251 explained above, the insertion hole portions 251A may be arranged in positions where the respective electric terminals 253A are close to one another.

### (Eighth Example)

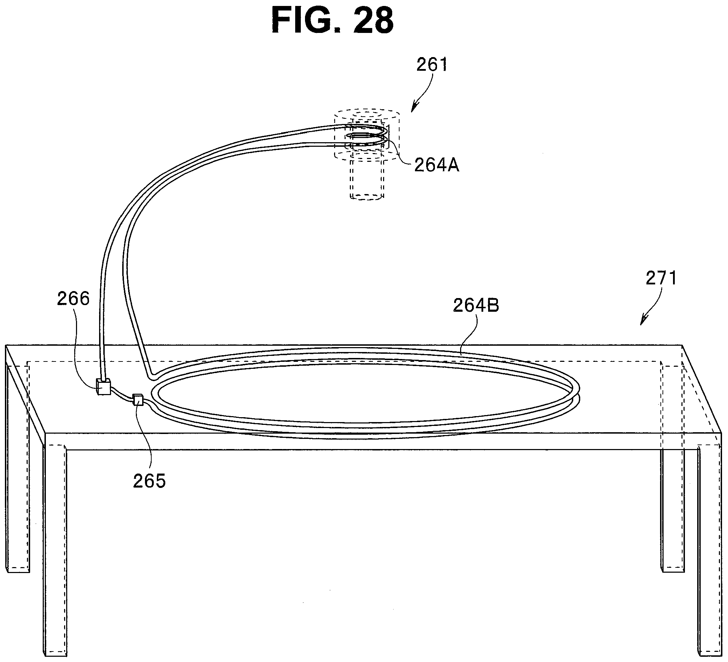

Fig. 28 relates to an eighth example.

Note that, in the present example, detailed explanation concerning portions including configurations and the like same as those in any one of the first to seventh examples is omitted. Portions including configurations and the like different from those in all of the first to seventh examples are mainly explained.
Fig. 28 is a diagram showing a configuration of a power transmission coil unit according to the eighth example.

As shown in Fig. 28, the power transmission coil unit in the present example is configured by connecting in series an intra-trocar power transmission coil 264A incorporated in a trocar 261, an intra-bed power transmission coil 264B incorporated in a bed 271 on which a subject to be treated with a medical instrument such as a bipolar electric knife can be laid, a power transmission capacitor 265, and an electric connection member 266.

The intra-trocar power transmission coil 264A and the intra-bed power transmission coil 264B are wound and connected such that generating directions of magnetic fields coincide with each other (or are not reversed).

Further, in the power transmission coil unit in the present example, inductance of the intra-trocar power transmission coil 264A, inductance of the intra-bed power transmission coil 264B, and capacitance of the power transmission capacitor 265 are respectively set such that the power transmission coil unit has a predetermined resonance frequency (e.g., 13.56 MHz).

With the power transmission coil unit including the configuration explained above, it is possible to generate a magnetic field in a wide range when power supply is performed making use of the magnetic field resonance phenomenon.

Note that, according to the respective examples explained above, the coil unit including the coil and the capacitor electrically connected in series is not limitedly configured to resonate in series. A coil unit including a coil and a capacitor electrically connected in parallel may be configured to resonate in parallel.

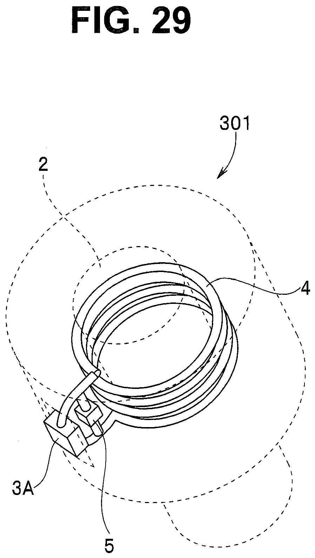

Fig. 29 is a diagram showing an example of a configuration in a case in which a power transmission coil unit capable of resonating in parallel is provided in a trocar.

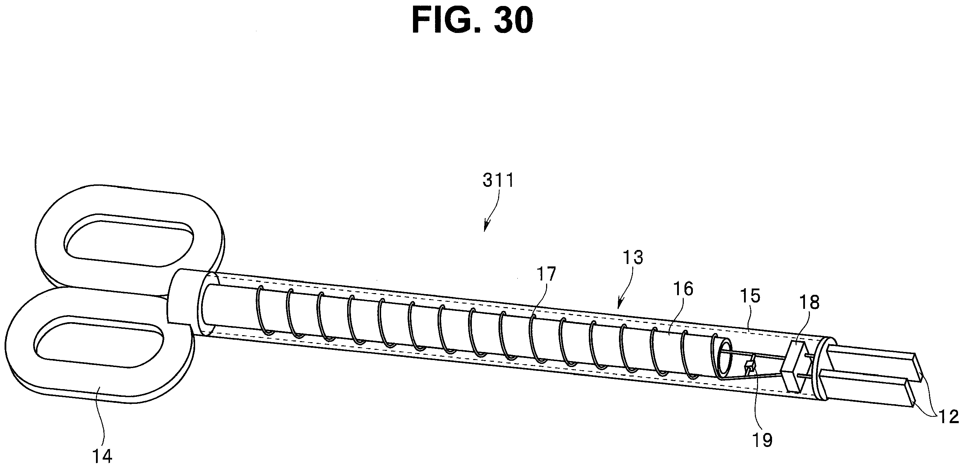

Fig. 30 is a diagram showing an example of a configuration in a case in which a power reception coil unit capable of resonating in parallel is provided in a bipolar electric knife.

More specifically, for example, as shown as a trocar 301 in Fig. 29, in a trocar, a power transmission coil unit may be provided in which the power transmission coil 4 and the power transmission capacitor 5 are connected to the electric terminal 3A electrically in parallel and inductance of the power transmission coil 4 and capacitance of the power transmission capacitor 5 are respectively set such that the power transmission coil unit has a predetermined parallel resonance frequency that coincides with a resonance frequency of the power reception coil unit incorporated in the bipolar electric knife 11 or the like.

For example, as shown as a bipolar electric knife 311 in Fig. 30, in a bipolar electric knife, a power reception coil unit may be provided in which the power reception coil 17 and the power reception capacitor 19 are connected to the conversion circuit 18 electrically in parallel and inductance of the power reception coil 17 and capacitance of the power reception capacitor 19 are respectively set such that the power reception coil unit has a predetermined parallel resonance frequency that coincides with a resonance frequency of the power transmission coil unit incorporated in the trocar 1 or the like.

Further, in the respective coil units, i.e., the power transmission coil unit, the power reception coil unit, and the relay coil unit, a coil unit configured to resonate in serial resonance and a coil unit configured to resonate in parallel resonance may be concurrently used.

More specifically, for example, the trocar 1 including the power transmission coil unit configured to resonate in serial resonance and the bipolar electric knife 311 including the power reception coil unit configured to resonate in parallel resonance may be concurrently used.

In the respective examples explained above, the respective coil units (the power transmission coil unit, the power reception coil unit, and the relay coil unit) is not limitedly configured by connecting one set of a coil and a capacitor in series. For example, the respective coil units may be configured using stray capacitance of the coil itself instead of the capacitor.

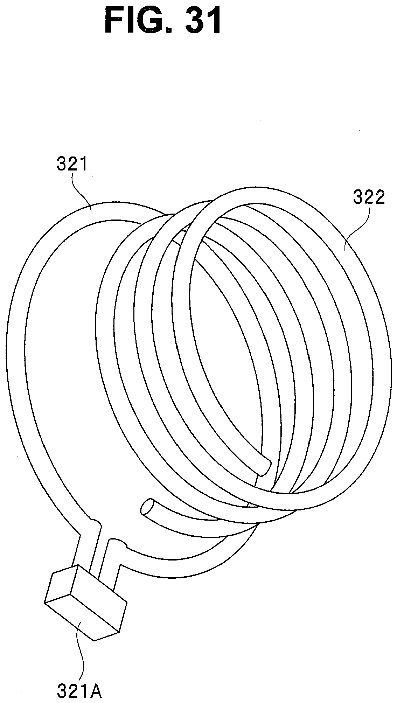

In the respective examples explained above, the electric terminal is not limitedly attached to the power transmission coil of the power transmission coil unit. For example, as shown in Fig. 31, electric power from a power supply device (not shown in the figure) may be supplied to an electric terminal 321A provided in a closed loop coil 321. Further, the closed loop coil 321 and a power transmission coil 322 may be respectively arranged to be separated by a space in which the electromagnetic induction phenomenon can be generated. Fig. 31 is a diagram showing an example of a configuration capable of being incorporated in the power transmission coil unit.

With the configuration explained above, electric power received wirelessly via the electric terminal 321A and the closed loop coil 321 can be transmitted from the power transmission coil 322.

In the respective examples explained above, the power transmission capacitor of the power transmission coil unit may be configured to be provided on the inside of the power supply device 21.

The respective examples explained above are also applicable to a medical instrument other than the bipolar electric knife as long as the medical instrument is inserted into the trocar and used. More specifically, even if, for example, an endoscope, a monopolar electric knife, an ultrasound treatment instrument, and the like are used instead of the bipolar electric knife, the respective examples explained above can be carried out.

In the respective examples explained above, for example, by providing a power storage device such as a battery in a medical instrument including a power reception coil unit, after electric power received in the power reception coil unit is stored in the power storage device, electric power corresponding to a state of use of the medical instrument may be supplied from the power storage device.

In the respective embodiments explained above, electric power received in the power reception coil unit of the bipolar electric knife may be directly supplied to the treatment electrodes not via the conversion circuit.

In the respective examples explained above, an impedance matching circuit may be provided in any one of the power supply device, the trocar, or the conversion circuit of the bipolar electric knife. With such a configuration, by controlling the impedance matching circuit according to, for example, a change in a position and a posture between the power transmission coil and the power reception coil or a change in a load state in the medical instrument including the power reception coil unit, it is possible to optimize transmission efficiency in performing power supply making use of the magnetic field resonance phenomenon.

## Claims

1. A medical wireless power supply system comprising:
a trocar (211,221);
a plurality of medical instruments (11,71,81,161,311) including a cylindrical insertion portion (13,73,83,163) insertable into an insertion hole (212,222) of the trocar (211,221);
a plurality of power transmission coils (214M, 214N, 224M, 224N) provided in a state in which at least a part thereof is covered with a nonconductive member in the vicinity of the insertion hole (212,222) of the trocar (211,221);
a plurality of power transmission coil units provided in the trocar (211,221), the power transmission coil units each including a respective one of the plurality of power transmission coils (214M, 214N, 224M, 224N) and configured to be capable of transmitting electric power supplied from the trocar (211,221) to the medical instruments (11,71,81,161,311) by respectively resonating at predetermined resonance frequencies differing from each other;
a power reception coil (17,77,87,167) in each of the plurality of medical instruments (11,71,81,161,311) provided in a state in which at least a part thereof is covered with a nonconductive member on an inside of the insertion portion (13,73,83,163); and
a power reception coil unit provided in each of the medical instruments (11,71,81,161,311), each power reception coil unit including the power reception coil (17,77,87,167) and configured to be capable of receiving electric power transmitted from the trocar (211,221) by resonating at a predetermined resonance frequency that coincides with a respective one of the predetermined resonance frequencies of the plurality of power transmission coil units.

2. The medical wireless power supply system according to claim 1, wherein winding axes of the power transmission coils (214M, 214N, 224M, 224N) and an insertion axis of the insertion hole (212,222) are parallel.

3. The medical wireless power supply system according to claim 1, wherein the power transmission coils (214M, 214N, 224M, 224N) are provided to generally cover a portion ranging from a vicinity of an opening portion on an inlet side of the insertion hole (212,222) to a vicinity of an opening portion on an outlet side on an inside of the trocar (211,221).

4. The medical wireless power supply system according to claim 1, wherein winding axes of the power reception coils (17,77,87,167) and a major axis of the insertion portion (13,73,83,163) are parallel.

5. The medical wireless power supply system according to claim 1, wherein the power reception coils (17,77,87,167) are provided in a portion ranging from a distal end side to a rear end side of the insertion portion(13,73,83,163).

6. The medical wireless power supply system according to claim 1, wherein the power reception coils (17,77,87,167) are provided in a position arranged near an opening portion on an inlet side of the insertion hole (212,222) when the insertion portion (13,73,83,163) is inserted through the insertion hole (212,222).

7. The medical wireless power supply system according to claim 1, wherein one or more relay coil units (166A) configured to be capable of resonating at the predetermined resonance frequency are provided on an inside of the insertion portion (163).

8. The medical wireless power supply system according to claim 1, wherein the trocar (211,221) is configured such that a conductive partition member formed to have sufficiently large width and height with respect to a diameter and height of the plurality of power transmission coils (214M, 214N, 224M, 224N) can be attached to and detached from the trocar (211,221).

9. The medical wireless power supply system according to claim 1, wherein, on an inside of the trocar (211,221), a conductive partition member having a shape capable of generally covering outer circumference portions of the plurality of power transmission coils (214M, 214N, 224M, 224N) and formed to include an insulating portion in a part of an outer circumference is provided.

## Patentansprüche

1. Drahtloses medizinisches Stromversorgungssystem mit:
einem Trokar (211, 221);
einer Mehrzahl von medizinischen Instrumenten (11, 71, 81, 161, 311), die einen zylindrischen, in ein Einführungsloch (212, 222) des Trokars (211, 221) einführbaren Einführungsabschnitt (13, 73, 83, 163) umfassen;
einer Mehrzahl von Stromübertragungsspulen (214M, 214N, 224M, 224N), die in einem Zustand vorliegen, in dem wenigstens ein Teil davon mit einem nichtleitenden Element in der Nähe des Einführungslochs (212, 222) des Trokars (211, 221) bedeckt ist.;
einer Mehrzahl von Stromübertragungsspuleneinheiten im Trokar (211, 221), wobei jede der Stromübertragungsspuleneinheiten jeweils eine aus der Mehrzahl von Stromübertragungsspulen (214M, 214N, 224M, 224N) umfasst und für die Übertragung von elektrischem Strom ausgelegt ist, der den medizinischen Instrumenten (11, 71, 81, 161, 311) von dem Trokar (211, 221) durch Schwingung bei vorbestimmten, voneinander unterschiedlichen Resonanzfrequenzen zugeführt wird;
einer Stromaufnahmespule (17, 77, 87,167) in jeder der Mehrzahl von medizinischen Instrumenten (11, 71, 81, 161, 311), die in einem Zustand vorliegen, in dem wenigstens ein Teil davon mit einem nichtleitenden Element auf der Innenseite des Einführungsabschnitts (13, 73, 83, 163) bedeckt ist; und
einer Stromaufnahmespuleneinheit in jeder der medizinischen Instrumente (11, 71, 81, 161, 311), wobei jede Stromaufnahmespuleneinheit eine Stromaufnahmespule (17, 77, 87,167) umfasst und für die Aufnahme von von dem Trokar (211, 221) übertragenen elektrischem Strom ausgelegt ist durch Schwingung bei einer vorbestimmten Resonanzfrequenz, die mit einer jeweiligen vorbestimmten Resonanzfrequenz von den Resonanzfrequenzen der Mehrzahl von Stromübertragungsspuleneinheiten zusammenfällt.

2. Drahtloses medizinisches Stromversorgungssystem nach Anspruch 1, bei dem die Windungsachsen der Stromübertragungsspulen (214M, 214N, 224M, 224N) und die Einführungsachse des Einführungslochs (212, 222) parallel sind.

3. Drahtloses medizinisches Stromversorgungssystem nach Anspruch 1, bei dem die Stromübertragungsspulen (214M, 214N, 224M, 224N) so vorgesehen sind, dass sie im Allgemeinen einen Abschnitt bedecken, der von einem Bereich des Öffnungsabschnitts auf der Eingangsseite des Einführungslochs (212, 222) bis zu einem Bereich des Öffnungsabschnitts auf der Ausgangsseite im Inneren des Trokars (211, 221) reicht.

4. Drahtloses medizinisches Stromversorgungssystem nach Anspruch 1, bei dem die Windungsachsen der Stromaufnahmespulen (17, 77, 87, 167) und eine Hauptachse des Einführungsabschnitts (13, 73, 83, 163) parallel sind.

5. Drahtloses medizinisches Stromversorgungssystem nach Anspruch 1, bei dem die Stromaufnahmespulen (17, 77, 87, 167) in einem Abschnitt vorgesehen sind, der von einer Seite des distalen Endes bis zu einer Seite des rückwärtigen Endes des Einführungsabschnitts (13, 73, 83, 163) reicht.

6. Drahtloses medizinisches Stromversorgungssystem nach Anspruch 1, bei dem die Stromaufnahmespulen (17, 77, 87, 167) in einer Position vorgesehen sind, die in der Nähe eines Öffnungsabschnitts auf der Eingangsseite des Einführungslochs (212, 222) angeordnet ist, wenn der Einführungsabschnitt (13, 73, 83, 163) durch das Einführungsloch (212, 222) eingeführt wird.

7. Drahtloses medizinisches Stromversorgungssystem nach Anspruch 1, bei dem eine oder mehrere Relaisspulen (166A), die für eine Schwingung bei einer vorbestimmten Resonanzfrequenz ausgelegt sind, auf der Innenseite des Einführungsabschnitts (163) vorgesehen sind,.

8. Drahtloses medizinisches Stromversorgungssystem nach Anspruch 1, bei dem der Trokar (211, 221) so ausgelegt ist, dass ein leitfähiges Trennelement von ausreichend großer Breite und Höhe hinsichtlich des Durchmessers und der Höhe der Mehrzahl von Stromübertragungsspulen (214M, 214N, 224M, 224N) an dem Tokar (211, 221) angebracht und von ihm entfernt werden kann.

9. Drahtloses medizinisches Stromversorgungssystem nach Anspruch 1, bei dem auf der Innenseite des Trokars (211, 221) ein leitfähiges Trennelement vorgesehen ist, dass so geformt ist, dass es im Allgemeinen die äußeren Umfangsabschnitte der Mehrzahl von Stromübertragungsspulen (214M, 214N, 224M, 224N) bedeckt und so gebildet ist, dass es einen isolierenden Abschnitt in einem Teil des äußeren Umfangs umfasst.

## Revendications

1. Système d'alimentation électrique médical sans fil comprenant :
un trocart (211, 221) ;
une pluralité d'instruments médicaux (11, 71, 81, 161, 311) incluant une partie d'introduction cylindrique (13, 73, 83, 163) pouvant être introduite dans un trou d'introduction (212, 222) du trocart (211, 221) ;
une pluralité de bobines de transmission d'énergie (214M, 214N, 224M, 224N) disposées dans un état dans lequel au moins une partie de celles-là est recouverte d'un élément non conducteur à proximité du trou d'introduction (212, 222) du trocart (211, 221) ;
une pluralité d'unités de bobine de transmission d'énergie disposées dans le trocart (211, 221), les unités de bobine de transmission d'énergie incluant chacune une bobine respective de la pluralité de bobines de transmission d'énergie (214M, 214N, 224M, 224N) et configurées pour être capables de transmettre de l'énergie électrique apportée depuis le trocart (211, 221) aux instruments médicaux (11, 71, 81, 161, 311) en résonant respectivement à des fréquences de résonance prédéterminées différant les unes des autres ;
une bobine de réception d'énergie (17, 77, 87, 167) dans chaque instrument de la pluralité d'instruments médicaux (11, 71, 81, 161, 311) disposée dans un état dans lequel au moins une partie de celle-là est recouverte d'un élément non conducteur sur un intérieur de la partie d'introduction (13, 73, 83, 163) et
une unité de bobine de réception d'énergie disposée dans chacun des instruments médicaux (11, 71, 81, 161, 311), chaque unité de bobine de réception d'énergie incluant la bobine de réception d'énergie (17, 77, 87, 167) et étant configurée pour être capable de recevoir l'énergie électrique transmise depuis le trocart (211, 221) en résonant à une fréquence de résonance prédéterminée qui coïncidé avec une fréquence respective des fréquences de résonance prédéterminées de la pluralité d'unités de bobine de transmission d'énergie.

2. Système d'alimentation électrique médical sans fil selon la revendication 1, dans lequel des axes d'enroulement des bobines de transmission d'énergie (214M, 214N, 224M, 224N) et un axe d'introduction du trou d'introduction (212, 222) sont parallèles.

3. Système d'alimentation électrique médical sans fil selon la revendication 1, dans lequel les bobines de transmission d'énergie (214M, 214N, 224M, 224N) sont fournies pour recouvrir généralement une partie partant des alentours d'une partie d'ouverture sur un côté entrée du trou d'introduction (212, 222) jusqu'aux alentours d'une partie d'ouverture sur un côté sortie d'un intérieur du trocart (211, 221).

4. Système d'alimentation électrique médical sans fil selon la revendication 1, dans lequel des axes d'enroulement des bobines de réception d'énergie (17, 77, 87, 167) et un axe majeur de la partie d'introduction (13, 73, 83, 163) sont parallèles.

5. Système d'alimentation électrique médical sans fil selon la revendication 1, dans lequel des bobines de réception d'énergie (17, 77, 87, 167) sont disposées sur une partie s'étendant du côté extrémité distale à un côté extrémité arrière de la partie d'introduction (13, 73, 83, 163).

6. Système d'alimentation électrique médical sans fil selon la revendication 1, dans lequel les bobines de réception d'énergie (17, 77, 87, 167) sont disposées dans une position agencée près d'une partie d'ouverture sur un côté entrée du trou d'introduction (212, 222) quand la partie d'introduction (13, 73, 83, 163) est introduite dans le trou d'introduction (212, 222).

7. Système d'alimentation électrique médical sans fil selon la revendication 1, dans lequel une ou plusieurs unités de bobine de relais (166A) configurées pour être capables de résonner aux fréquences de résonance prédéterminées sont disposées sur un intérieur de la partie d'introduction (163).

8. Système d'alimentation électrique médical sans fil selon la revendication 1, dans lequel le trocart (211, 221) est configuré de telle sorte qu'un élément de séparation conducteur formé pour avoir une largeur et une hauteur suffisamment grandes par rapport à un diamètre et à une hauteur de la pluralité de bobines de transmission d'énergie (214M, 214N, 224M, 224N) peut être attaché au trocart (211, 221) et en être détaché.

9. Système d'alimentation électrique médical sans fil selon la revendication 1, dans lequel, sur un intérieur du trocart (211, 221), un élément de séparation conducteur ayant une forme capable de recouvrir généralement des parties de circonférence extérieure de la pluralité de bobines de transmission d'énergie (214M, 214N, 224M, 224N) et formé pour inclure une partie isolante dans une partie d'une circonférence extérieure est prévu.
